Europäisches Patentamt

European Patent Office

Office européen des brevets

(19)

(11) **EP 1 669 063 A1**

(12) **EUROPÄISCHE PATENTANMELDUNG**

(43) Veröffentlichungstag:
 **14.06.2006 Patentblatt 2006/24**

(51) Int Cl.:
 *A61K 9/70* *(2006.01)* *A61K 31/381* *(2006.01)*

(21) Anmeldenummer: **06005770.0**

(22) Anmeldetag: **29.07.2003**

(84) Benannte Vertragsstaaten:
 **AT BE BG CH CY CZ DE DK EE ES FI FR GB GR HU IE IT LI LU MC NL PT RO SE SI SK TR**
 Benannte Erstreckungsstaaten:
 **LT LV**

(30) Priorität: **30.07.2002 DE 10234673**

(62) Dokumentnummer(n) der früheren Anmeldung(en) nach Art. 76 EPÜ:
 **03750403.2 / 1 492 517**

(71) Anmelder: **SCHWARZ PHARMA AG**
 **40789 Monheim (DE)**

(72) Erfinder:
 • **Breitenbach, Armin**
  **51371 Leverkusen (DE)**
 • **Wolff, Hans-Michael**
  **40789 Monheim (DE)**

(74) Vertreter: **Hildebrand, Steven**
 **Schwarz Pharma AG**
 **Alfred-Nobel-Straße 10**
 **D-40789 Monheim (DE)**

Bemerkungen:
 Diese Anmeldung ist am 21 - 03 - 2006 als Teilanmeldung zu der unter INID-Kode 62 erwähnten Anmeldung eingereicht worden.

(54) **TTS zur Verabreichung von Rotigotin**

(57) Die vorliegende Patentanmeldung betrifft ein transdermales therapeutisches System (TTS) zur Verabreichung von Rotigotin mit einer wirkstoffhaltigen Schicht die einen hohen Rotigotinanteil enthält, so dass nach dem Auftrag des TTS auf der Haut des Patienten Rotigotin über einen Zeitraum von mindestens 5 Tagen in einer steadystate Fluxrate von 100-500 µg/Stunde durch die Haut abgegeben wird.

EP 1 669 063 A1

**Beschreibung**

Die Erfindung betrifft ein Transdermales Therapeutisches System (TTS) enthaltend eine Rotigotin-haltige Klebermatrix, dadurch gekennzeichnet, dass die Klebermatrix einen heißschmelzfähigen Haftkleber enthält, in dem der Wirkstoff Rotigotin ((-)-5,6,7,8-Tetrahydro-6-[propyl[2-(2-thienyl)ethyl)amino]-1-naphtol) dispergiert, teilweise oder vollständig gelöst ist.

Ferner betrifft die Erfindung ein Verfahren zur Herstellung eines TTS umfassend eine Rotigotin als Wirkstoff enthaltende Klebermatrix, dadurch gekennzeichnet, dass die Bestandteile der Klebermatrix vor dem Beschichten und Laminieren bei Temperaturen zwischen 70 und 200°C und bevorzugt zwischen 120 und 160°C lösungsmittelfrei geschmolzen und homogenisiert werden.

Schließlich betrifft die Patentanmeldung die Verwendung von Rotigotin zur Herstellung der Klebermatrix eines TTS im Heißschmelzverfahren.

Aus dem Stand der Technik sind verschiedene TTS zur Verabreichung von Rotigotin bekannt.

WO 94-07468 offenbart ein System, das ein Wirkstoffsalz in einer Zweiphasenmatrix enthält. Die Zweiphasenmatrix besteht aus einem hydrophoben Polymer mit einem darin dispergierten Silikat zur Aufnahme des hydrophilen Arzneistoffsalzes, wobei zusätzlich organische Lösungsmittel verwendet werden. Die Herstellung der Matrix erfolgt durch Trocknen der Dispersion bei 70°C. Der Gehalt an Rotigotin in der Matrix beträgt 2- 5 Gew%.

Dieses System weist jedoch eine Reihe von Nachteilen auf:

(1) Die Herstellung ist mehrstufig und aufwendig. Das Wirkstoffsalz muß in Wasser bzw. in einem wässrigen Lösungsmittelgemisch gelöst, dann mit dem Silikat gemischt werden, dann mit einem Emulgator vermengt werden, um die wässrige Lösung schließlich mit dem in einem organischen Lösungsmittel -üblicherweise Heptan, Ethylacetat oder Toluol- gelösten Polymer, z.B. in einem Silikonkleber, zu emulgieren. Die resultierende Emulsion ist schwierig zu handhaben.

(2) Es werden organische Lösungsmittel verwendet, die bei der Herstellung des TTS wieder vollständig entfernt werden müssen, um eine ausreichende Lagerstabilität und reproduzierbare Freisetzungeigenschaften des TTS zu gewährleisten und Hautreizungen zu verhindern. Dadurch erhöhen sich die Herstellkosten. Das Verfahren ist bis zur Stufe der wirkstoffhaltigen Klebermasse diskontinuierlich.

(3) Der Umgang mit organischen Lösungsmitteln erfordert erhöhte Sicherheitsvorkehrungen, um eine Belastung von Umwelt und mit der TTS-Produktion befassten Mitarbeitern zu verhindern. Vorrichtungen zur Lösungsmittel-Wiedergewinnung/Abscheidung, Schutzmaßnahmen für das Personal und Maßnahmen zur Entsorgung der Lösungsmittel sind kostenaufwendig.

(4) Die Wirkstoffbeladung ist zum einen begrenzt durch die Löslichkeit des Rotigotins im jeweiligen Lösungsmittelsystem. Zum anderen findet beim Entfernen der Lösungsmittel während der Herstellung eine Aufkonzentrierung statt, bei der es zu einer Übersättigung des Matrixsystems mit Wirkstoff und zu einer unerwünschten Kristallbildung kommen kann. Auch hierdurch ist die maximale Menge an Wirkstoff, die sich in die Matrix einarbeiten läßt, begrenzt. Eine niedrige Wirkstoffbeladung wiederum begrenzt die Freisetzungskapazität der Matrix pro Zeiteinheit und/oder ihre funktionale Lebenszeit infolge vorzeitiger Wirkstoffentleerung.

(5) Die in einem Herstellungsschritt erreichbare Matrixdicke ist auf etwa 100 $\mu$m Dicke (entspricht ca 100 g/m$^2$) begrenzt, um eine vollständige Entfernung des zur Herstellung erforderlichen Lösungsmittels beim Trocknungsprozeß zu gewährleisten. Sind Klebermatrices von mehr als etwa 100 $\mu$m Dicke erforderlich, so müssten diese in mehreren Teilschichten aufgebaut werden. Dies ist jedoch aufwendig und erhöht die Kosten.

(6) Das im Pflaster verbleibende Silicat bzw. Siliziumdioxid stellt eine Diffusionsbarriere für den Wirkstoff dar, die die Freigabe des Wirkstoffes negativ beeinflussen kann. Des weiteren wird die Wasseraufnahme des Pflasters beeinflußt. Porenbildung durch Herauslösen wasserlöslicher Matrixbestandteile an der Grenzfläche zur Haut können zu einer schlecht kontrollierbaren Freisetzung des Wirkstoffes führen.

Die WO 99/49852 beschreibt ein TTS mit Rotigotin in Form der freien Base enthaltend ein Haftklebesystem, welches auf Acrylat oder Silikon basiert. Bei der Herstellung beider Systeme werden ebenfalls Lösungsmittel verwendet, die bei der Herstellung wieder entfernt werden müssen, verbunden mit den oben unter den Punkten (2) -(5) beschriebenen Nachteilen und Einschränkungen.

Im Hinblick auf die Beladung und Freisetzung von Rotigotin haben die beiden in der WO 99/49852 beschriebenen Matrices zudem die folgenden Nachteile:

Silikonmatrices: Ausgehend von einer wirkstoffhaltigen Emulsion oder Lösung ist eine Beladung der Matrix mit ca. 15 Gew% Rotigotin möglich. Der Beladbarkeit von Siliconmatrices mit Wirkstoff sind daher Grenzen gesetzt. Eine Erhöhung der Rotigotin-Beladung z.B. zur Herstellung von Mehrtages-Pflastern ist nur durch den Auftrag weiterer Matrixschichten möglich, was jedoch mehrere Arbeitsgänge erfordert und die Herstellung aufwendiger und teurer macht.

Acrylatmatrices: Acrylatmatrices können mittels Lösungsmittelbeschichtung mit bis zu ca. 40 Gew% Rotigotin beladen werden. Dem höheren Aufnahmevermögen der Matrix für Rotigotin steht bei diesen Matrices jedoch ein vermindertes Abgabevermögen an die Haut wegen eines im Vergleich zu Silikonsystemen ungünstigeren Wirkstoffverteilungskoeffizienten gegenüber. Um ausreichende Plasmaspiegel Rotigotin aus solchen Systemen zu erzielen, sind zum einen sehr hohe Beladungen erforderlich. Zum anderen verbleiben relativ hohe Wirkstoffanteile nach Anwendung im Pflaster, was die Herstellungskosten solcher Systeme erhöht und aus Gründen der Arzneimittelsicherheit unerwünscht ist.

Ziel der vorliegenden Erfindung war es daher, ein Rotigotin TTS zur Verfügung zu stellen, das die mit der Verwendung von Lösungsmitteln verbundenen Nachteile und Beschränkungen vermeidet. Insbesondere sollte das Rotigotin-TTS eine größtmögliche Flexibilität bei der Beladung mit Rotigotin, auch in größeren Mengen, aufweisen und das Rotigotin in therapeutisch relevanter Menge freisetzen.

Die oben genannten Probleme wurden gelöst, indem erstmals ein TTS mit einer Rotigotinhaltigen Klebermatrix zur Verfügung gestellt wurde, die dadurch gekennzeichnet ist, dass die Klebermatrix im Heißschmelzverfahren hergestellt wurde, so dass die Klebermatrix einen heißschmelzfähigen Haftkleber enthält, in dem der Wirkstoff Rotigotin ((-)-5,6,7,8-Tetrahydro-6-[propyl[2-(2-thienyl)ethyl)amino]-1-naphtol) dispergiert, teilweise oder vollständig gelöst ist.

**Abbildungen:**

Abbildungen 1 a und b zeigen Vergleiche der Permeation von Rotigotin durch Mäusehaut (HMS) aus Heißschmelz-Silikon-TTS mit lösungsmittel-basiertem Silikon-TTS. Abbildung 1 a zeigt die Freisetzung aus TTS bei einem Rotigotin-Gehalt von jeweils 9 Gew%. Abbildung 1 b zeigt den Einfluß der höheren Beladung mit Rotigotin auf die Permeation von Rotigotin durch Mäusehaut aus Heißschmelz TTS.

Abbildung 2 zeigt den Einfluß des Wachsgehalts auf die Permeation von Rotigotin durch Mäusehaut aus Heißschmelz-Silikon-TTS bei konstanter Wirkstoffbeladung von 9 Gew%.

Abbildungen 3a und b zeigen den Einfluß der Rotigotin-Beladung auf Permeation von Rotigotin durch Mäusehaut aus silikon-basiertem Heißschmelz-TTS in Gegenwart von 15% Wachs (3a) bzw. 5% Wachs (3b).

Abbildung 4 zeigt den Einfluß des Matrixgewichts auf die Permeation von Rotigotin durch Mäusehaut aus silikon-basiertem Heißschmelz-TTS.

Abbildungen 5 a und b zeigen den Einfluß des Gehalts an innerer-Phase-Komponente (PVP) auf die kumulative (5a) bzw. lineare (5b) Permeation von Rotigotin durch Mäusehaut aus Heißschmelz-TTS. Abbildung 5c zeigt den Einfluß der Konzentration von PEO auf die kumulative Permeation von Rotigotin durch Humanhaut aus Silikon-Heißschmelz-TTS.

Abbildung 6 a zeigt die kumulative Permeation von Rotigotin durch Humanhaut aus Heißschmelz-Silikon-TTS über 72 Stunden in Vergleich zu lösungsmittel-basiertem Silikon-TTS. Abbildung 6 b zeigt die kumulative Permeation von Rotigotin durch Humanhaut aus Heißschmelz-Silikon-TTS über 7 Tage. Abbildung 6 c zeigt die kumulative Permeation von Rotigotin durch Humanhaut aus Heißschmelz-Silikon-TTS mit 5 % Ozokerit oder 5 % Ceresin über 7 Tage.

Abbildung 7 zeigt die kumulative Permeation von Rotigotin durch Mäusehaut aus Heißschmelz-TTS mit verschiedenen heißschmelzfähigen Klebern.

Abbildung 8 zeigt die kumulative Permeation von Rotigotin durch Mäusehaut aus im Extruder hergestellten silikon-basierten Heißschmelz-TTS mit verschiedenen innere-Phase-Komponenten und einem Rotigotin-Gehalt von 9%.

Abbildung 9 zeigt die kumulative Permeation von Rotigotin durch Mäusehaut aus im Extruder hergestellten, EVA-basierten Heißschmelz-TTS mit verschiedenem Rotigotin-Gehalt. Abbildung 9a zeigt die kumulative Permeation von Rotigotin durch Humanhaut aus EVA-basiertem Heißschmelz-TTS im Vergleich zum Iösemittelbasierten Silikon-TTS.

Abbildung 10 zeigt ein Beispiel eines TTS-Aufbaus mit einer wirkstoffhaltigen Klebermatrix (1), einer für die Bestandteile der Klebermatrix inerten Rückschicht (2) und einer vor Gebrauch zu entfernenden Schutzfolie (3).

**Beschreibung der Erfindung:**

Überraschenderweise wurde festgestellt, daß sich Rotigotin im Heißschmelzverfahren ausgezeichnet verarbeiten lässt, bei kurzzeitiger Erhitzung auf Temperaturen bis mindestens 160°C stabil ist, sich in den durch Heißschmelzverfahren hergestellten Matrices homogen einarbeiten lässt und aus den Heißschmelzmatrices kontinuierlich und in therapeutisch gewünschter Rate freigesetzt wird.

Insbesondere wurde von den Erfindern überraschend festgestellt, dass das oxidationsempfindliche Rotigotin im Heißschmelzverfahren stabil bleibt, selbst wenn es auf Temperaturen um 160°C erhitzt wird. Obwohl Rotigotin bei höheren Temperaturen in sauerstoffhaltiger Atmosphäre zur oxidativen Zersetzung neigt, erweist es sich in der heißen Klebstoffschmelze als überraschend stabil und liegt in der Matrix in einem Reinheitsgrad von regelmäßig über 98%, im allgemeinen über 99% vor (gemessen bei 220 nm und 272 nm per HPLC; siehe Tabellen 2, 3 und 4).

Bevorzugt wird das Rotigotin in fester Form in die homogenisierte Matrixschmelze eingebracht, so dass das Rotigotin erst in der heißen Matrix geschmolzen wird. Nach kurzer Homogenisierung wird die Rotigotin-haltige Klebermatrix wieder abgekühlt, so dass das Rotigotin im Allgemeinen für weniger als 5 Minuten, bevorzugt weniger als 4, 3 oder sogar weniger als 1 Minute, thermischer Belastung ausgesetzt wird. Danach liegt Rotigotin in der erstarrten Schmelze vor. Während dieses Prozesses ist das Rotigotin von kritischen Umwelteinflüssen (Licht, Sauerstoff) weitgehend abgeschirmt.

Die auf diese Weise durch Heißschmelzverfahren hergestellten TTS erlauben eine hohe Beladung des Rotigotins mit bis zu über 40 Gew% bezogen auf das Matrixgewicht.

Insgesamt weisen die erfindungsgemäß durch Heißschmelzverfahren hergestellten TTS eine Reihe von Vorteilen gegenüber den aus dem Stand der Technik bekannten lösungsmittelbasierten TTS auf:

- Da das Rotigotin direkt in die Klebstoffschmelze eingebracht werden kann, entfallen die Lösungsmittel-bedingten Probleme bei der Verarbeitung höherer Wirkstoffkonzentrationen. Als Folge können in einfacher Weise wesentlich höhere Konzentrationen (bis über 40 Gew%) Rotigotin in das TTS eingebracht werden, als dies im Lösungsmittel-basierten Verfahren auf Silikonbasis der Fall ist, wo Rotigotin-Konzentrationen von über ca. 15 Gew% nicht mehr als Lösung einarbeitbar sind. Dies ermöglicht das Einbringen überraschend hoher Rotigotinmengen auch bei relativ dünnen Matrices und in nur einem Arbeitsgang.

- Die Schichtdicke kann in einem weiten Bereich variiert werden. Somit ist auch die Herstellung von Matrices mit einem Gewicht von über 100 g/m$^2$ und auch über 200 g/m$^2$ in einem Arbeitsgang problemlos möglich. Daraus folgt, dass in Kombination mit der höheren Rotigotin-Konzentration ein Rotigotin-Gehalt in der TTS-Matrix mit bis zu 8 mg/cm$^2$ oder sogar darüber erreichbar sind. Im Gegensatz dazu ist mit dem im Lösungsmittel-basierten Verfahren hergestellten Silikon-TTS eine Rotigotin-Beladung über ca. 1,5 mg/cm$^2$ in einem Herstellschritt nicht möglich.

- Einsatz, Entfernen, Rückgewinnung oder Nachverbrennung von organischen Lösungmitteln sowie die damit verbundenen Sicherheitsvorkehrungen bei der TTS-Herstellung entfallen.

- Die Heißschmelztechnologie erlaubt eine kontinuierliche Produktion der TTS Matrix beginnend mit der Einwaage ihrer Einzelkomponenten bis hin zur Laminierung. Eine solche Prozeßführung bietet im Wesentlichen folgende Vorteile:

  ♦ Die Prozeßzeiten werden erheblich verkürzt.

  ♦ Die Chargengröße kann über die Betriebsdauer der Produktionsanlage festgelegt werden. Ein Wechsel auf größere Anlagen mit den damit verbundenen Scale-up Problemen und/oder zusätzlichem Validierungsaufwand wird dadurch vermieden.

- Eine GMP-konforme Produktion kann auf kompakten Anlagen mit geringem Platzbedarf erfolgen.

- Durch die Verwendung eines geeigneten Weichmachers, z.B. Wachs und/oder die optionale Inkorporation einer inneren Phase kann zudem eine Retardierung der Freisetzung von Rotigotin aus der Klebermatrix erzielt werden. Bei entsprechender Ausgestaltung des TTS ermöglicht dies die Herstellung von TTS, die Rotigotin über einen Zeitraum von mehreren Tagen, z.B. für 5, 6 oder 7 Tage kontinuierlich in therapeutisch relevanter Menge freisetzen.

Ein Gegenstand der Erfindung ist daher ein transdermales therapeutisches System (TTS) enthaltend eine Rotigotin-haltige Klebermatrix, dadurch gekennzeichnet, dass die Klebermatrix einen heißschmelzfähigen Haftkleber enthält.

Ein weiterer Gegenstand der Erfindung ist ein transdermales therapeutisches System (TTS) enthaltend eine Rotigotin-haltige Klebermatrix, dadurch gekennzeichnet, dass die Klebermatrix einen heißschmelzfähigen Haftkleber enthält, in dem der Wirkstoff Rotigotin ((-)-5,6,7,8-Tetrahydro-6-[propyl[2-(2-thienyl)ethyl)amino]-1-naphtol) dispergiert, teilweise oder vollständig gelöst ist.

In einer anderen Ausführungsform der Erfindung enthält die heißschmelzfähige Klebermatrix statt Rotigotin ein Rotigotin-Prodrug, d.h. eine Verbindung, z.B. ein Ester, die im Körper des Patienten, z.B. durch Esterasen im Blut oder der Haut in therapeutisch effektiver Menge zu Rotigotin gespalten bzw. metabolisiert wird. Bevorzugt sollte das Prodrug dabei soviel Rotigotin freisetzen, dass eine therapeutisch effektive steady-state Konzentration Rotigotin im Plasma erreicht wird. Solche Konzentrationen liegen bevorzugt zwischen 0,05 und 20 ng/ml, besonders bevorzugt zwischen 0,1 und 10 ng/ml und ganz besonders bevorzugt zwischen 0,2 und 5 ng Rotigotin/ml Plasma.

Ein weiterer Gegenstand der Erfindung ist ein TTS mit einer im Heißschmelzverfahren hergestellten Rotigotin-haltigen Klebermatrix, wobei die Klebermatrix hergestellt wurde, indem das Rotigotin in geschmolzener oder, bevorzugt, in fester Form in die lösungsmittelfreie, 70-200°C heiße Schmelze der Klebermatrix eingebracht wurde. Bevorzugt wird das Rotigotin in eine lösungsmittelfreie Schmelze eingebracht, die 100-170°C, besonders bevorzugt 120-160°C und ganz besonders bevorzugt 130-150°C heiß ist und die innerhalb von 5 Minuten, bevorzugt innerhalb von 3 Minuten, 2 Minuten oder besonders bevorzugt innerhalb von höchstens 1 Minute nach Zugabe des Rotigotins verarbeitet und abgekühlt wird.

Unter "transdermales therapeutisches System" wird dabei eine pharmazeutische Formulierung oder Vorrichtung verstanden, die zur transdermalen Wirkstoffverabreichung durch Säugerhaut, insbesondere durch Humanhaut, in therapeutisch relevanter Menge geeignet ist.

Unter "Heißschmelzverfahren" wird dabei verstanden, dass zum Schmelzen der Bestandteile der Klebermatrix, insbesondere zum Schmelzen des heißschmelzfähigen Haftklebers sowie der optional vorhandenen inneren Phase, thermische Energie eingesetzt wird, so daß auf den Einsatz von Lösungsmitteln zur Herstellung der Klebermatrix verzichtet werden kann. Unter "Heißschmelzverfahren" wird in dieser Anmeldung auch eine Verfahrensvariante subsumiert, in dem bei Temperaturen unterhalb des Rotigotin-Schmelzpunktes gearbeitet wird, so daß das Rotigotin in fester Form in der Klebstoffschmelze vorliegt.

Unter "lösungsmittelfrei" wird in dieser Anmeldung verstanden, dass zur Herstellung der Klebermatrix keine Lösungsmittel verwendet werden, die im Verlaufe des Herstellverfahrens wieder entfernt werden müssen.

Unter "heißschmelzfähiger Haftkleber" wird dabei ein Haftkleber verstanden, der beim Aufbringen auf die Haut druckempfindlich ist und der sich bei den Verfahrenstemperatu ren von 70°C bis 200°C, bevorzugt von 100°C bis 170°C, besonders bevorzugt von 120°C bis 160°C und ganz besonders bevorzugt bei Temperaturen zwischen 130°C und 150°C im Heißschmelzverfahren verarbeiten lässt. Dabei kann der "heißschmelzfähige Haftkleber" aus einem Haftkleber oder einer Mischung verschiedener Haftkleber bestehen, die per se heißschmelzfähig sind. Alternativ kann der "heißschmelzfähige Haftkleber" auch eine Mischung eines Haftklebers mit einem geeigneten Weichmacher umfassen.

Bevorzugt weisen solche heißschmelzfähigen Haftkleber bei 160°C und insbesondere bei Temperaturen zwischen 130°C und 150°C eine dynamische Viskosität von höchstens 150 Pa.s, bevorzugt höchstens 120 Pa.s, besonders bevorzugt weniger als 100 Pa.s, ganz besonders bevorzugt weniger als 80 Pa.s oder sogar weniger als 60 Pa.s auf.

Haftkleber, die nicht per se heißschmelzfähig sind, sind beispielsweise handelsübliche Silikonkleber. Silikonkleber sind bei den oben genannten Verarbeitungstemperaturen zu viskos, d.h. haben eine dynamische Viskosität von über 150 Pa.s.

In der Patentliteratur wurden verschiedene Verfahren beschrieben, die Viskosität von Silikonklebern durch Beimischung von geeigneten Zusätzen (Weichmachern) heißschmelzfähig zu machen. Beispiele für solche Weichmacher für Silikone sind Glycerolmonolaurat oder Laurylactetat, wie in EP 835 136 beschrieben, Wachse der Formel R-C (O)-OR' wie in EP 360 467 beschrieben, Alkylmethylsiloxanwachse wie in EP 524 775 beschrieben, siloxierte Polyetherwachse, wie in EP 663 431 beschrieben oder organische Wachse, wie in US RE 36 754 beschrieben.

Die Weichmacher werden dem Silikonkleber im Allgemeinen in einer Menge von 1-30 Gew% bezogen auf das Gesamtgemisch der heißschmelzfähigen Klebermischung zugesetzt. Bevorzugte Weichmacher sind organische Wachse, wie in US RE 36 754 beschrieben, z.B. Ozokerit, Ceresin, Paraffin, Candelila, Carnauba, Bienenwachs oder Mischungen dieser Wachse, wobei Ozokerit und Ceresin ganz besonders bevorzugt werden.

Vorgefertigte heißschmelzfähige Silikonhaftkleber, speziell Mischungen aus Silikonhaftklebern mit Ceresin oder Ozokerit können bei Dow Corning, Michigan, bezogen werden. Durch den Zusatz von 10 Gew% Ceresin zu einem Silikon-Haftkleber gelang es beispielsweise, die dynamische Viskosität des resultierenden Haftklebergemischs bei einer Verarbeitungstemperatur von 160°C von über 150 Pa.s auf unter 50 Pa.s zu senken. Eine solche silikonbasierte Haftklebermischung kann in einem Temperaturbereich von 100°C bis 200°C und insbesondere im Bereich zwischen 120°C und 160°C gut im Heißschmelzverfahren verarbeitet werden.

Überraschenderweise wurde festgestellt, daß heißschmelzfähige Silikonhaftkleber ausgezeichnet zur transdermalen Verabreichung von Rotigotin geeignet sind.

Ein Gegenstand der Erfindung ist daher ein transdermales therapeutisches System (TTS) enthaltend eine Rotigotin-haltige Klebermatrix, dadurch gekennzeichnet, dass die Klebermatrix einen heißschmelzfähigen Haftkleber enthält, in dem der Wirkstoff Rotigotin ((-)-5,6,7,8-Tetrahydro-6-[propyl[2-(2-thienyl)ethyl)amino]-1-naphtol) dispergiert ist, wobei der heißschmelzfähige Haftkleber eine geeignete Mischung aus einem silikonbasierten Haftkleber und mindestens einem Weichmacher enthält.

Ein weiterer Aspekt der Erfindung ist ein TTS umfassend eine Klebermatrix, die umfasst:

(a) 50-99 Gew% einer Haftklebermischung bestehend aus

(i) 70-99 Gew% eines amin-resistenten Silikonklebers
(ii) 1-30 Gew% eines geeigneten Weichmachers

(b) 1-40 Gew% Rotigotin oder ein Rotigotin-Prodrug.

In einer bevorzugten Ausführungsform der Erfindung besteht der besagte silikonbasierte heißschmelzfähige Haftkleber

(a) zu 70-99 Gew% aus einem amin-resistenten Silikonkleber und

(b) zu 1-30 Gew%, bevorzugt 3-15 Gew% und ganz bevorzugt zu 4-10 Gew% aus organischem Wachs, welches besonders bevorzugt ausgewählt wird aus der Gruppe Ozokerit, Ceresin, Paraffin, Candelilla, Carnauba, Bienenwachs oder Mischungen dieser Wachse, wobei Ozokerit und insbesondere Ceresin ganz besonders bevorzugt werden.

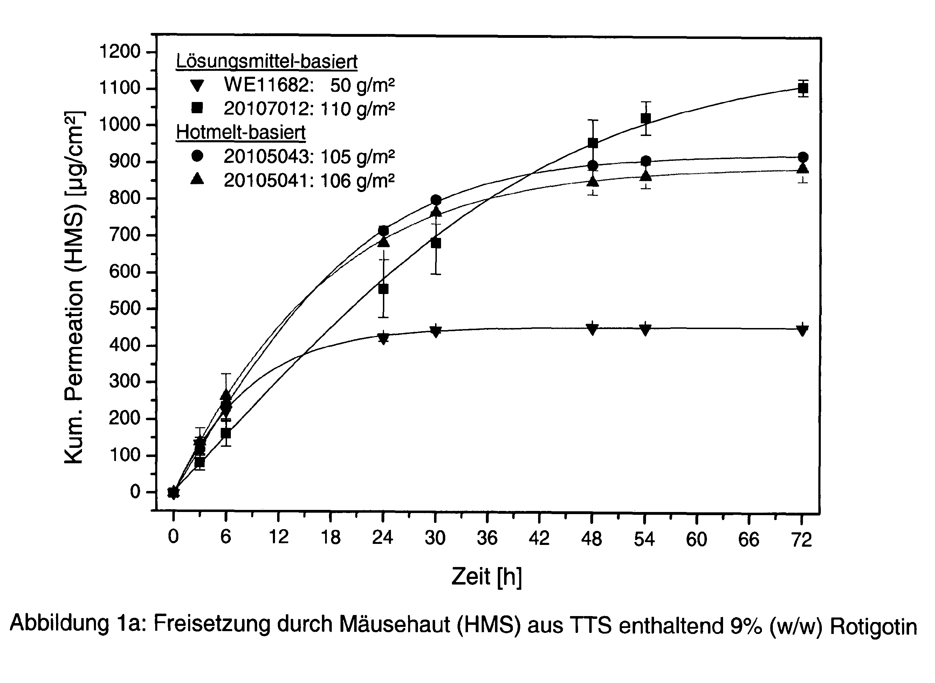

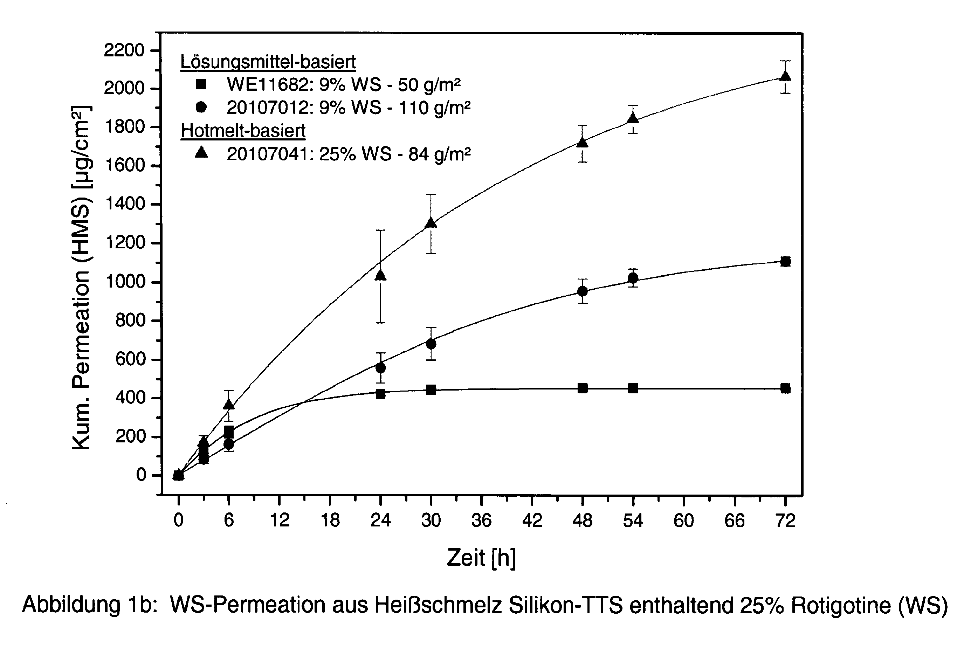

Abbildung 1 a zeigt, dass ein solch einfach aufgebautes silikonbasiertes Heißschmelz-TTS zu in-vitro Permeationsraten von Rotigotin führt, die mit denen des aus dem Stand der Technik bekannten, therapeutisch effektiven, Lösungsmittel-basierten Silikon-TTS vergleichbar sind.

Abbildung 1 b zeigt, dass bei entsprechend hoher Beladung des erfindungsgemäßen Heißschmelz-Silikon-TTS in-vitro Fluxraten erreichbar sind, die deutlich über den Raten liegen, die mit den aus dem Stand der Technik bekannten, klinisch effektiven Lösungsmittel-basierten Silikonpflastern liegen.

Unter "Heißschmelz-TTS" wird in dieser Patentanmeldung ein TTS verstanden, dessen Klebermatrix im Heißschmelzverfahren, das heißt durch lösungsmittelfreies Aufschmelzen der heißschmelzfähigen Haftkleber, sowie gegebenenfalls weiterer Bestandteile, hergestellt wurde.

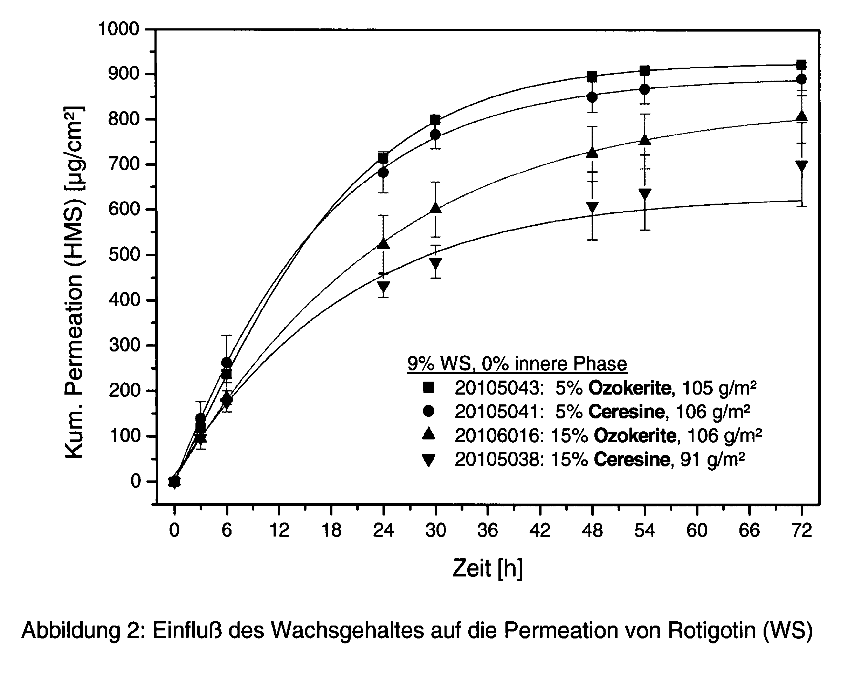

Überraschenderweise wurde gefunden, dass der Zusatz von Wachs, insbesondere von organischen Wachsen, wie Ceresin oder Ozokerit, einen Einfluß auch auf die in-vitro Permeation durch Mäusehaut von Rotigotin aus dem Heißschmelz-Silikon-TTS hat. Wie aus Abbildung 2 ersichtlich, sinkt die Permeationsrate des Rotigotins mit steigender Konzentration von Wachs. Dies ist mit einer partiellen Verteilung von Rotigotin im Wachs und einer damit verbundenen Retardierungswirkung zu erklären.

Diese Eigenschaft des Wachses hat Bedeutung insbesondere bei der Entwicklung eines TTS, das zur Anwendung über mehrere Tage, z.B. über 7 Tage, konzipiert ist. Ein solches Mehrtagespflaster erfordert eine hohe Beladung mit Rotigotin, mit der Gefahr der überschießenden Rotigotin-Freisetzung zu Beginn der Applikationsphase ("Dose-Dumping"). Es ist daher sinnvoll, eine die Wirkstofffreisetzung steuernde Komponente in das TTS einzuarbeiten. Dies kann eine an der Unterseite der Matrix aufgebrachte, die Wirkstofffreisetzung steuernde Membran sein. Eine solche Membran führt aber zu erhöhten Materialkosten und macht den TTS-Aufbau aufwendiger. Daher wäre es wünschenswert, anstelle der Verwendung einer zusätzlichen Membran geeignete retardierende Komponenten in die Matrix einzuarbeiten.

Da nun überraschenderweise durch den Wachsanteil in der Matrix die Wirkstofffreisetzung retardiert wird, steuert die Variation des Wachsgehaltes nicht nur die dynamische Viskosität des Haftklebers, sondern bietet auch die überraschende zusätzliche Option der Regulierung der Wirkstoftreisetzung:

Die dynamische Viskosität des Silikonhaftklebers nimmt mit steigendem Wachsgehalt zunächst bis zu einem Wachsgehalt von ca. 5 Gew% stark ab, sinkt dann aber nur noch geringfügig. Bei einem Wachsgehalt von 4-10 Gew% ist daher die dynamische Viskosität des Silikonhaftklebers auf eine für die Heißschmelz-Verarbeitung geeignete Viskosität eingestellt, wobei gleichzeitig der Einfluss auf die Freisetzung des Rotigotins gering ist. Bei höheren Wachskonzentrationen wird zusätzlich ein Retardierungseffekt erreicht.

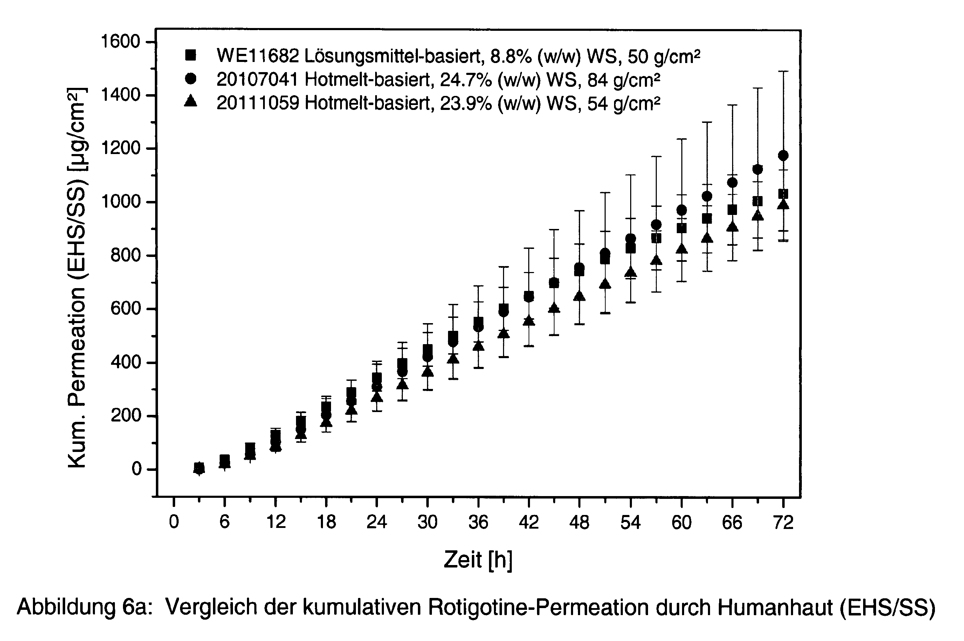

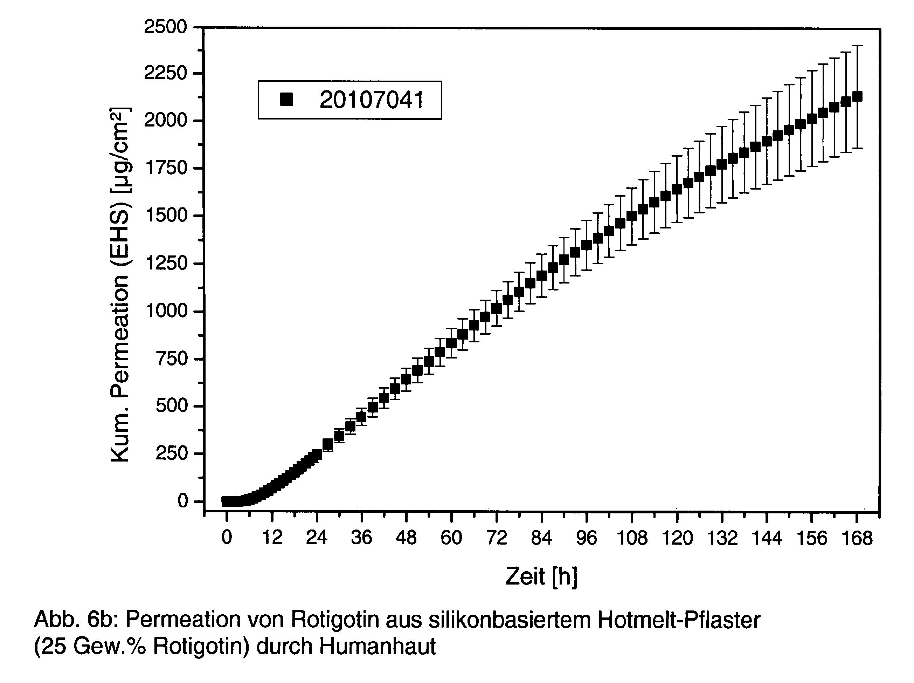

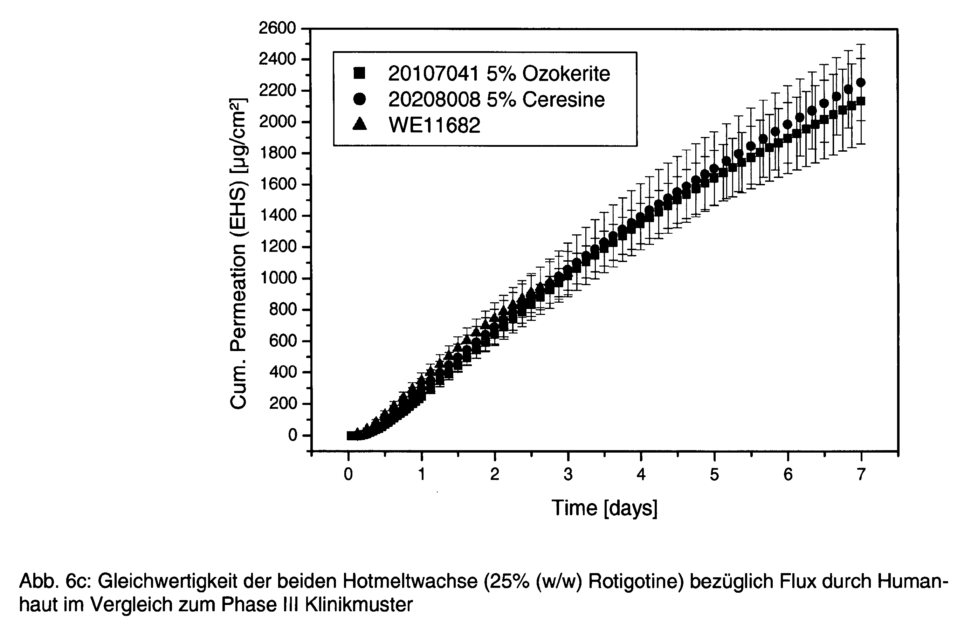

Die Abbildungen 6a und 6c zeigen, dass bei Zugabe von 5 Gew% organischem Wachs zu einem mit vergleichsweise hohen Rotigotin-Mengen beladenen (hier ca. 25 Gew%) TTS Fluxraten durch Humanhaut erzielt werden, die mit denen vergleichbar sind, die mit einem geringer beladenen (9 Gew%) Lösungsmittel-TTS erzielt werden. Dies erlaubt die Herstellung von TTS mit längerer Wirkstofffreisetzung, z.B. über 7 Tage (Abb. 6b und 6c).

Auch der Einfluß des Wachses auf die rheologischen Eigenschaften des TTS ist überraschend. Wird ein organisches Wachs als Weichmacher für einen Silikonhaftkleber verwendet, sinkt die dynamische Viskosität des Haftklebergemisches bei höheren Temperaturen, so dass das silikonbasierte Klebergemisch ausgezeichnet im Heißschmelzverfahren verarbeitbar ist. Gleichzeitig bleiben die rheologischen Eigenschaften des Silikons, z.B. die Kohäsivität, bei Raumtemperatur aber unerwarteterweise weitgehend unbeeinflusst, so daß typische Probleme heißschmelzfähiger Haftkleber, wie z.B. kalter Fluss auf der Haut des Patienten, nicht auftreten.

Als Silikone kommen grundsätzlich die in der Pflastertechnologie bekannten Silikonhaftkleber in Frage. Bevorzugte Silikonhaftkleber sind amin-resistente, druck-sensitive Polyorganosiloxankleber. Silikonhaftkleber stellen in den meisten Fällen Polydimethylsiloxane dar, allerdings können prinzipiell statt Methylgruppen auch andere organische Reste, wie z.B. Ethyl oder Phenylgruppen vorhanden sein. Aminresistente Silikonhaftkleber zeichnen sich im Allgemeinen dadurch aus, dass sie keine oder nur wenige freie Silanolfunktionen enthalten, da die Si-OH-Gruppen alkyliert wurden. Solche Kleber sind in der EP 180 377 beschrieben. Besonders bevorzugte Kleber sind Kondensate oder Mischungen von Silikonharzen und Polyorganosiloxanen, wie beispielsweise in US RE 35 474 beschrieben.

Solche Silikon-Haftkleber sind kommerziell erhältlich und werden beispielsweise von Dow Corning als Bio-PSA Q7-4300 oder Bio-PSA Q7-4200 vertrieben. Heißschmelzfähige Silikonhaftkleber, bestehend aus Mischungen von PSA 7-4300 mit organischen Wachsen wie Ozokerit oder Ceresin, sind ebenfalls bei Dow Corning erhältlich.

Der Wirkstoff Rotigotin kann in Mengen von 1 bis über 40 Gew% bezogen auf das Gewicht der gesamten Kleberschicht vorliegen, wobei Rotigotin als Salz oder freie Base vorliegen kann. Bevorzugt liegt Rotigotin in der Klebermatrix als freie Base vor. Alternativ kann das Rotigotin auch in Form eines Prodrugs, z.B. als Rotigotin-Ester oder Rotigotin-Carbamat, vorliegen.

Im Gegensatz zu den Lösungsmittel-basierten Silikonhaftklebern, die einen Wirkstoffanteil von höchstens 15 Gew% enthalten, ist bei den Heißschmelz-TTS eine Beladung der Klebstoffmatrix mit deutlich höheren Mengen Rotigotin ohne zusätzlichen technischen Aufwand möglich. Dies erlaubt eine größere Flexibilität in der Einstellung der Permeationsrate sowie der Freisetzungsdauer des Heißschmelz TTS.

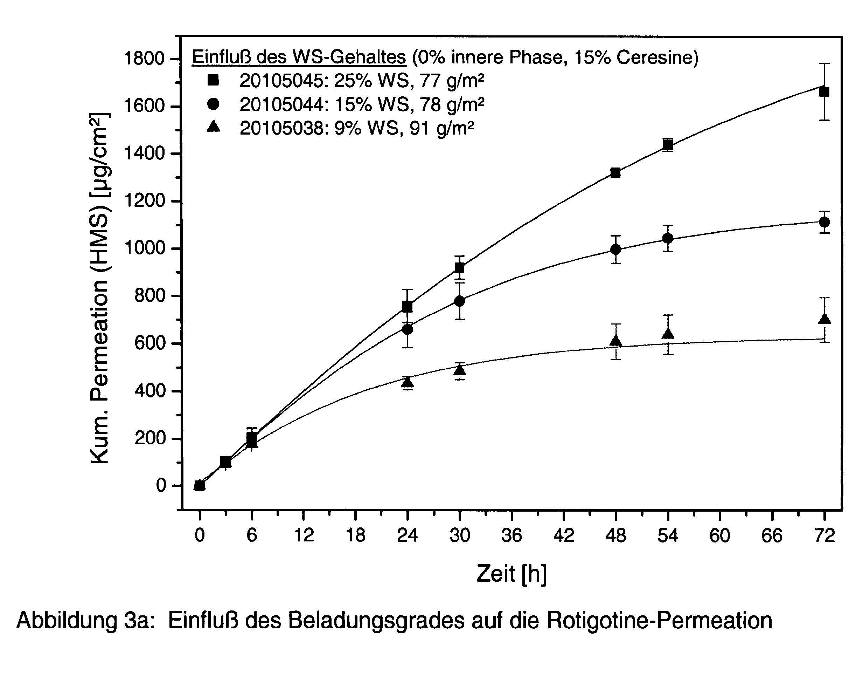

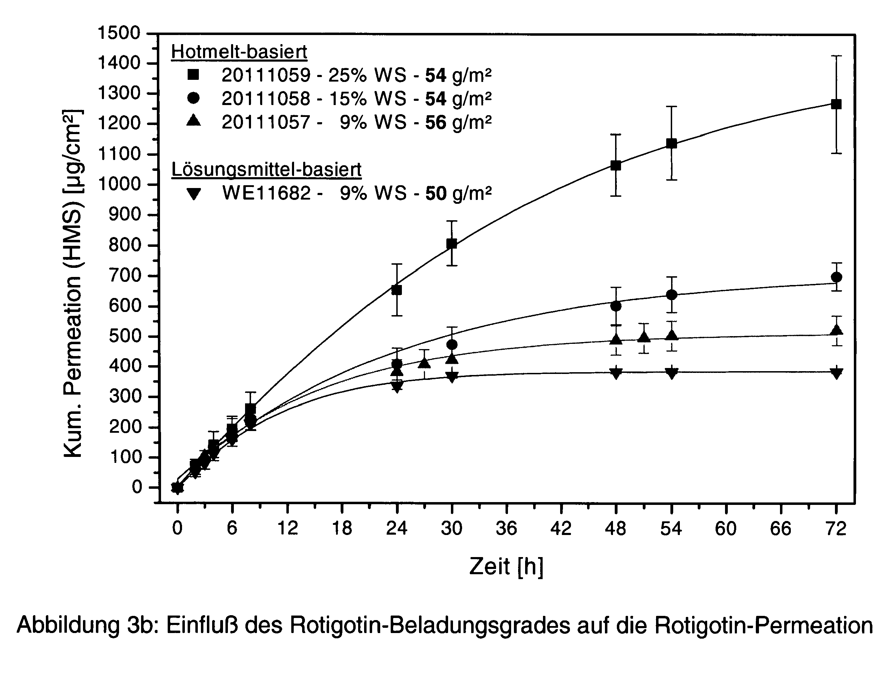

Wie in den Abbildung 3a und 3b am Beispiel der silikonbasierten Heißschmelz-TTS ersichtlich ist, kann mit einer höheren Rotigotin-Beladung sowohl eine höhere Fluxrate durch Säugerhaut als auch eine längere Dauer der Rotigotinfreisetzung erreicht werden. Bei Auftrag des TTS auf Humanhaut hat eine höhere Rotigotinbeladung insbesondere eine Verlängerung der Rotigotinfreisetzung zur Folge, während die Permeationsrate durch Humanhaut ab einem Anteil von etwa 8-9% Rotigotin nur noch wenig steigt.

Bevorzugte Konzentrationen von Rotigotin oder eines Rotigotin-Prodrugs in der Kleberschicht sind 4-40 Gew%, insbesondere 9-30 Gew% und ganz besonders 9-25 Gew% oder 15-25 Gew% und für 7-Tage-Pflaster 20-40 Gew% und insbesondere 25-35 Gew% bezogen auf das Gesamtgewicht der Kleberschicht.

In einem Kuheuter-Modell wurde überprüft, ob silikonbasierte Heißschmelz-TTS mit einer hohen Rotigotinbeladung (>15 Gew%) hautverträglich sind. Hierzu wurden die Zellvitalität sowie die $PGE_2$-Synthese nach Auftrag entsprechender Rotigotin-Heißschmelz-TTS (5 % Ceresin, 2 % PEO, 25 - 30 % Rotigotin) gemessen.

Während die Zellaktivität sich nach Auftrag des erfindungsgemäßen TTS nicht signifikant von der unbehandelten Haut unterschied, zeigte sich bezüglich der $PGE_2$-Synthese initial ein leichter Anstieg, der jedoch 5 Stunden nach dem Auftrag nicht mehr nachweisbar war. Im Vergleich zeigte sich nach der Behandlung der Haut mit einer 10 %igen SDS-Lösung signifikante Zelltetalität (über 50 % nach 5 h) und ein deutlicher Anstieg der $PGE_2$-Synthese (> 60 % nach 5 h) als Zeichen einer Entzündungsreaktion. Daraus kann gefolgert werden, dass die erfindungsgemäßen, zur mehrtägigen Rotigotin-Applikation geeigneten Heißschmelz-TTS trotz hoher Rotigotin-Beladung nicht zu signifikanter Hautirritation führen.

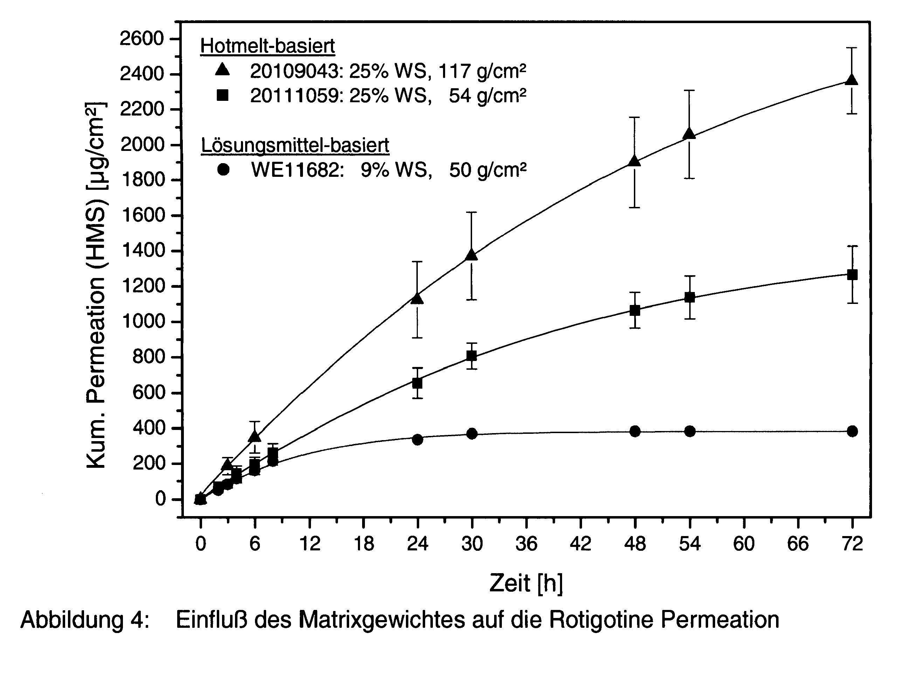

Als zusätzliches Element zur Steuerung der Freisetzungsrate und Dauer von Rotigotin steht die Variation der Beschichtungsdicke der Klebermatrix zur Verfügung. Abbildung 4 zeigt den Einfluss des Matrixgewichts auf die in vitro Permeation von Rotigotin durch Mäusehaut am Beispiel von Heißschmelz-Silikon-TTS.

Die Dicke der Klebermatrix kann in einem einzigen Arbeitsgang flexibel über einen weiten Bereich eingestellt werden, da die mit dem lösungsmittel-Verfahren verbundenen Einschränkungen der Schichtdicke wegfallen. Die Schichtdicken können zwischen 30 und 300 $\mu$m, bevorzugt zwischen 50 und 150 $\mu$m und besonders bevorzugt zwischen 50 und 120 $\mu$m liegen.

Die Klebermatrix der erfindungsgemäßen TTS hat bevorzugt ein Gewicht zwischen 30 und 300 $g/m^2$ und besonders bevorzugt zwischen 50 und 150 $g/m^2$ und ganz besonders bevorzugt zwischen 50 und 120 $g/m^2$; bei 7-Tagespflastern bevorzugt von 70-200 $g/m^2$ und besonders bevorzugt von 80 und 180 $g/m^2$ und zwischen 100 und 160 $g/m^2$.

Der bevorzugte Rotigotingehalt in der Matrix liegt je nach vorgesehener Auftragsdauer des TTS zwischen 0.4 $mg/cm^2$- 8 $mg/cm^2$.

Für ein 1 Tages-TTS ist die bevorzugte Beladung zwischen 0,4 und 1,5 $mg/cm^2$, besonders bevorzugt zwischen 0,4 und 0,8 $mg/cm^2$.

Die therapeutisch im Durchschnitt erforderliche Dosis bei Erwachsenen liegt bei etwa 6 mg Rotigotin/Tag. Für ein 7-Tage-Pflaster sind daher im Schnitt etwa 42 mg Wirkstoff pro TTS notwendig. Aus Sicherheitsgründen wird bei klinisch verwendeten transdermalen Systemen von einer im Mittel nur ca. 50-60%igen Ausschöpfung des TTS-Reservoirs ausgegangen. Daher sollte ein 7-Tage TTS bevorzugt mindestens 70 mg bis 84 mg Wirkstoff enthalten.

Bei einer bevorzugten TTS-Größe für ein 7-Tage Pflaster von 10-30 $cm^2$, besonders bevorzugt 15 - 25 $cm^2$ ergibt sich somit die folgende bevorzugte Rotigotinbeladung:

| Pflastergröße cm$^2$ | Mindest-Rotigotin-Gehalt mg/cm$^2$ |
|---|---|
| 10 | 7,0-8,4 |
| 15 | 4,7-5,6 |
| 20 | 3,5-4,2 |
| 25 | 2,8-3,4 |
| 30 | 2,3-2,8 |

Bei 7-Tagespflastern liegt der Rotigotin-Gehalt bzw. der Rotigotin-Prodrug-Gehalt somit bevorzugt zwischen ca. 2 mg/cm$^2$ und 8 mg/cm$^2$, besonders bevorzugt zwischen ca. 2,8 mg/cm$^2$ und 5,6 mg/cm$^2$ und ganz besonders bevorzugt zwischen ca. 3,1 und 5,6 mg/cm$^2$.

TTS zur Verabreichung von Rotigotin in therapeutisch relevanter Menge, die einen solch hohen Gehalt von Rotigotin aufweisen, sind aus dem Stand der Technik bisher nicht bekannt und wurden erst durch die variable Beladung und Beschichtungsdicke des Heißschmelz-TTS möglich. Die hohe Rotigotin-Beladungsrate von bis zu über 40 Gew% ermöglicht dabei auch bei einem 7-Tage TTS mit entsprechend hohem Rotigotingehalt die Herstellung relativ dünner Matrices von 80-200 $\mu$m, bevorzugt 80-180 $\mu$m, besonders bevorzugt 80-160 $\mu$m Schichtdicke.

Ein Gegenstand der vorliegenden Erfindung sind daher TTS zur Verabreichung von Rotigotin in therapeutisch relevanter Menge, dadurch charakterisiert, dass sie einen Rotigotingehalt in der Klebermatrix von mindestens 2,0 mg/cm$^2$, bevorzugt mindestens 2,8 mg/cm$^2$, besonders bevorzugt mindestens 3,1 mg/cm$^2$ oder mindestens 3,4 mg/cm$^2$ aufweisen. Bevorzugt werden dabei TTS enthaltend Matrices mit einer Rotigotin-Beladungsrate über 20 Gew% und einem Matrixgewicht von unter 200 g/m$^2$, z.B mit einem Gewicht zwischen 80 und 180 g/m$^2$ und besonders bevorzugt zwischen 80 und 160 g/m$^2$ (entspricht ca. 80-200 $\mu$m Schichtdicke).

Der Kleberschicht (auch als Klebermatrix bezeichnet) kann neben Rotigotin und der Haftklebermischung optional auch eine Komponente zugesetzt werden, die als innere Phase dient.

Diese innere-Phase-Komponente dient insbesondere als Lösungsvermittler und Kristallisationsinhibitor und trägt zu einer gleichmäßigen Verteilung des Wirkstoffs in der Klebermatrix bei. Die innere-Phase-Komponente dient weiterhin zur Erhöhung der Feuchtigkeitsaufnahme des Pflasters auf der Haut.

Zum Einsatz im Heißschmelzverfahren sind solche innere-Phase-Komponenten besonders geeignet, die bei Temperaturen unterhalb von 170°C eine dynamische Schmelzviskosität von maximal 150 Pa.s, bevorzugt kleiner als 120 Pa.s, 100 Pa.s und besonders bevorzugt von unter 80 Pa.s aufweisen.

Ist die dynamische Viskosität der innere-Phase-Komponente bei der gewünschten Verarbeitungstemperatur zu gering, so muss gegebenenfalls ein geeigneter Weichmacher, z.B. Glycerin, vorab zugesetzt werden. In einigen Fällen kann auch der Wirkstoff Rotigotin weichmachende Eigenschaften haben. Dies ist zum Beispiel bei Polyvinylpyrrolidon der Fall, so daß zur Eindosierung von PVP in einen Extruder eine Vorschmelze aus PVP und Rotigotin hergestellt werden kann.

Diese innere-Phase-Komponenten sind bevorzugt ausgewählt aus der Gruppe

(a) hydrophiler oder amphiphiler Polymere,
(b) hydrophiler oder amphiphiler Copolymere,
(c) Mischungen aus (a) und/oder (b) mit pharmazeutisch akzeptablen Weichmachern,
(d) Kondensate aus Glycerin mit Fettsäuren oder Polyolen,
(e) geeignete Mischungen aus den Bestandteilen (a)-(d)

Geeignete innere-Phase-Komponenten zur Verwendung im erfindungsgemäßen TTS können beispielsweise ausgewählt sein aus der Gruppe der Polysaccharide, substituierte Polysaccharide, Polyethylenoxide, Polyvinylacetate, Polyvinylpyrrolidone (PVP), PVP mit geeignetem Weichmacher, Polyethylenglycole, Polypropylenglycole, Acrylate, Copolymere aus Polyvinylpyrrolidon und (Poly)vinylacetat, Copolymere von Ethylen und Vinylacetat sowie Polyvinylalkoholen mit geeignetem Weichmacher, z.B. Glycerin.

Bevorzugte innere-Phase-Komponenten sind PVP, PVP mit Weichmacher, Polyethylenoxide (PEO), Polyvinylacetate (PVA) sowie Copolymere aus PVP und Vinylacetat.

Die innere-Phase-Komponente wird der Kleberschicht in einem Anteil von 0-40 Gew% bezogen auf das Gesamtgewicht der Kleberschicht zugesetzt. Bevorzugt werden 2-25 Gew % innere Phase-Komponente zugesetzt.

Überraschenderweise wurde gefunden, dass die innere-Phase Komponente bei konstanter Menge Wirkstoff und, gegebenenfalls, Weichmacher nicht nur die Löslichkeit des Rotigotins und damit die gleichmäßige Verteilung in der Matrix fördert, sondern mit steigender Menge auch zu einer Retardierung bzw. Linearisierung der Rotigotin-Freisetzung führen kann.

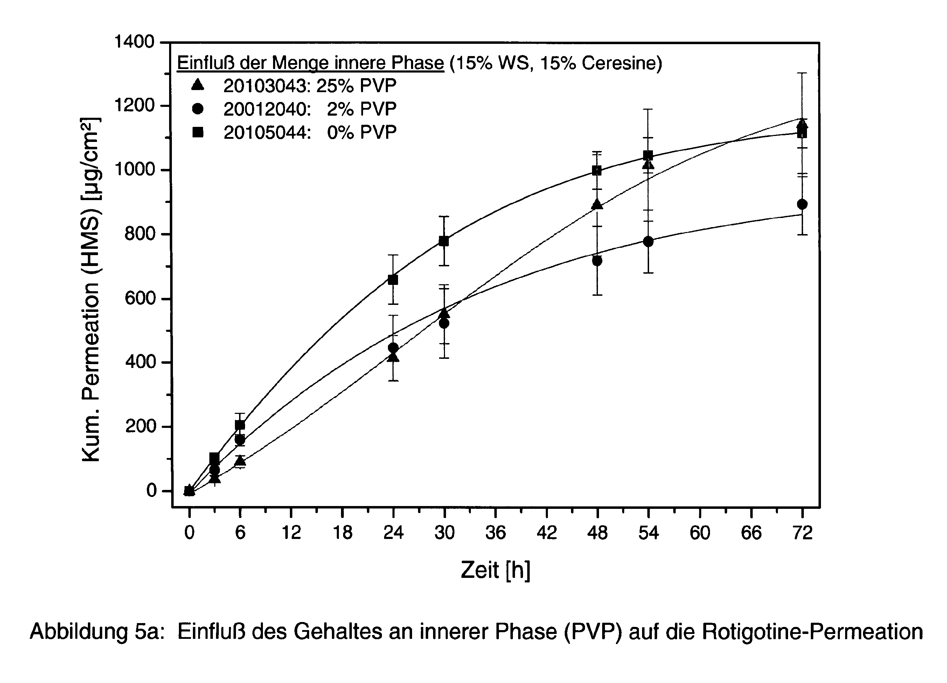

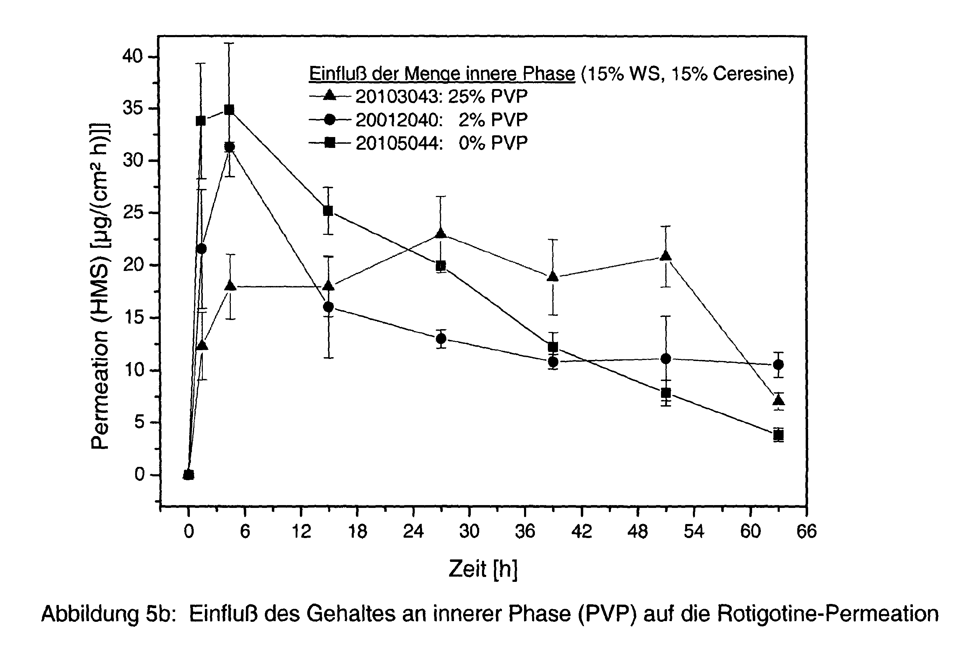

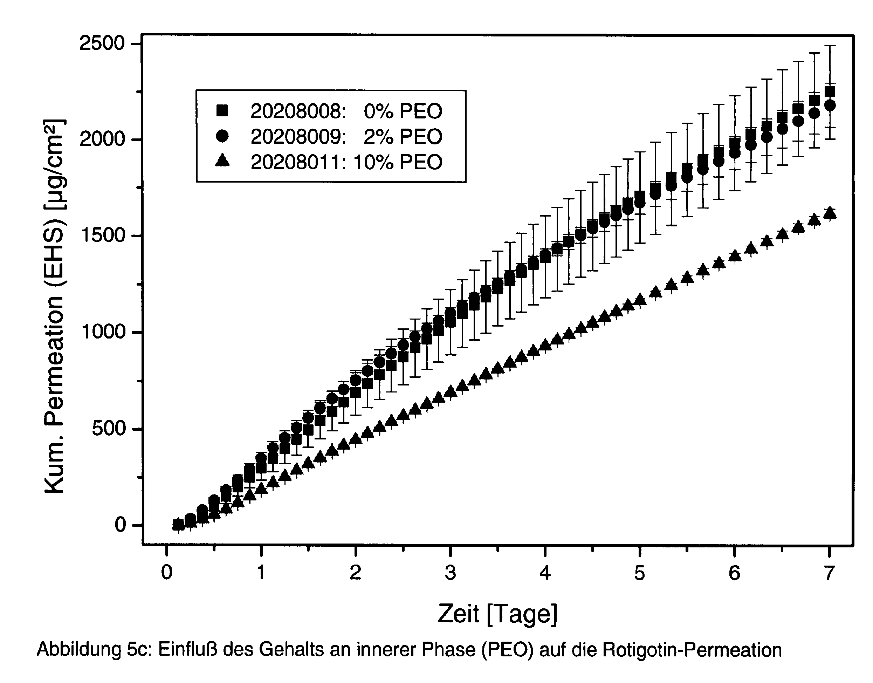

Abbildungen 5a und 5b zeigen am Beispiel des silikonbasierten Heißschmelz-TTS den Einfluß des Gehalts an PVP auf die in vitro Permeation von Rotigotin durch Mäusehaut. Mit steigendem PVP-Gehalt wird eine Linearisierung der Rotigotin-Permeation erreicht (Abb 5a), die auf eine signifikante Senkung der initialen Wirkstofffreisetzung zurückzuführen ist (Abb 5b). Abbildung 5 c zeigt den Einfluss verschiedener Konzentrationen PEO auf die Rotigotin-Permeation durch Humanhaut aus Silikon-Heißschmelz-TTS.

Ein solcher Retardierungseffekt der inneren Phase-Komponente kann z.B. bei Heißschmelz-TTS mit hoher Wirkstoffbeladung genutzt werden, um ein Pflaster bereitzustellen, das den Wirkstoff Rotigotin über einen längeren Zeitraum, z.B. für mindestens 3 Tage, mindestens 4, 5, 6 oder 7 Tage gleichmäßig in therapeutisch relevanter Menge abgibt.

Ausgehend von einer durchschnittlichen Tagesdosis von 6 mg Rotigotin ergibt sich eine erforderliche stündliche steady-state Fluxrate von 250 μg Rotigotin. Für ein TTS mit einer Fläche zwischen 10 und 30 cm$^2$ bedeutet dies eine erforderliche Fluxrate von 8,3-25 μg/cm$^2$/h.

In in-vitro Permeationsexperimenten an Humanhaut konnten mit dem erfindungsgemäßen silikonbasierten Heißschmelz-TTS bei einer Rotigotin-Beladung von ca. 23-25 Gew% und einem Pflastergewicht von 54-84 g/m$^2$, d.h. bei einem Rotigotingehalt von 1,2-2,1 mg/cm$^2$ Matrix, über einen Zeitraum von mindestens 3 Tagen kontinuierliche Fluxraten von 12-16 μg/cm$^2$/h erreicht werden (siehe Abbildung 6a).

Die Fluxrate bewegte sich dabei in der Größenordnung der klinisch relevanten Fluxrate des im Lösungsmittelverfahren hergestellten Vergleichs-TTS auf Silikonbasis. Während die Permeationskurve des eingesetzten Vergleichs-TTS nach ca. 48 Stunden wegen Erschöpfung des Wirkstoffreservoirs abknickt, waren die höher beladenen Heißschmelz TTS nach 72 Stunden noch nicht erschöpft.

Mit dem erfindungsgemäßen Heißschmelz-TTS konnte im verwendeten HumanhautModell, wie in Ausführungsbeispiel 9 beschrieben, bei einer Beladung mit Rotigotin von 25 Gew% und einem Matrixgewicht von 85 g/m$^2$ nach einer anfänglichen lag-Phase eine in-vitro steady-state Fluxrate durch Humanhaut von ca. 15 μg/cm$^2$/h über 7 Tage aufrechterhalten werden (Abbildung 6b). Dieses Ergebnis konnte in einem weiteren Versuch bestätigt werden, wobei sich die Wachse Ceresin und Ozokerit als gleichwertig erwiesen (Abb. 6c).

Ein Gegenstand der Erfindung ist somit ein TTS, das in der Klebermatrix als Wirkstoff Rotigotin oder ein Rotigotin-Prodrug in einer Menge von mindestens 20 Gew%, bevorzugt über 25 Gew% enthält, und in einem in vitro-Permeationstest mit humaner Haut, gemäß Ausführungsbeispiel 9, über einen Zeitraum von mindestens 5, 6 oder 7 Tagen zu einer kontinuierlichen Flußrate von mindestens 8 μg/cm$^2$/h, bevorzugt mindestens 10 μg/cm$^2$/h, führt.

Ein anderer Gegenstand der Erfindung ist ein Heißschmelz-TTS, das in der Kleberschicht Rotigotin als Wirkstoff in einer Menge von mindestens 20 Gew%, bevorzugt mindestens 25 Gew% enthält und in einem in vitro-Permeationstest mit humaner Haut, gemäß Ausführungsbeispiel 9, über einen Zeitraum von mindestens 7 Tagen zu einer kontinuierlichen Flußrate von mindestens 8 μg/cm$^2$/h führt.

Ferner wurde erstmals ein TTS zur Verfügung gestellt, das Rotigotin über einen Zeitraum von mindestens 5, 6 oder 7 Tagen mit einer stündlichen Fluxrate von 200-300 μg durch Säugerhaut, insbesondere durch Humanhaut, verabreichen kann.

Ein Aspekt der vorliegenden Erfindung ist daher ein TTS, vorzugsweise ein Heißschmelz-TTS, besonders bevorzugt ein silikonbasiertes Heißschmelz-TTS, das zur kontinuierlichen Verabreichung von Rotigotin über einen Zeitraum von mindestens 5, 6 oder 7 Tagen mit einer steady-state Fluxrate von 200-300 μg/Tag geeignet ist.

Unter dem Ausdruck "steady-state" wird in der vorliegenden Patentanmeldung ein Fließgleichgewicht verstanden, das sich nach einer initialen lag-Phase nach dem erstmaligen Auftragen der erfindungsgemäßen Vorrichtung einstellt.

Unter "steady-state Fluxrate" wird eine Fluxrate im Fließgleichgewicht verstanden, die sich nach der initialen lag Phase einstellt.

In einer bevorzugten Ausführungsform der Erfindung sind die in der Patentanmeldung genannten Fluxraten konstante Fluxraten.

Unter dem Ausdruck "konstante Fluxrate" wird in dieser Patentanmeldung eine steady-state Fluxrate verstanden, bei der Rotigotin in einer mittleren Fluxrate durch Humanhaut transportiert wird, die eine intraindividuelle Variabilität CV über die Zeit von maximal 30 %, bevorzugt maximal 20 % oder sogar maximal 10 % aufweist, wobei CV nach der Gleichung CV = (sd : x) x 100 % bestimmt wird (siehe Berechnung Cawello (ED) in "Parameters for Compartment-free Pharmacokinetics", Shaker Verlag, Aachen, 1999, Seite 112). Eine Tagesdosis wird dabei in einer mittleren Fluxrate von Tagesdosis:24 (mg/Stunde) mit einer CV von 30 % verabreicht. Für den Fachmann ist kar, dass sich eine konstante Fluxrate erst nach einer initialen Anflutungsphase ("lag-Phase") nach erstmaligem Auftrag der Vorrichtung einstellt. Die lag-Phase wird daher bei der Berechnung der konstanten Fluxrate nicht berücksichtigt.

Ein anderer Gegenstand der Anmeldung ist ein TTS zur transdermalen Verabreichung von Rotigotin, enthaltend eine wirkstoffhaltige Schicht, dadurch gekennzeichnet, dass

(a) die wirkstoffhaltige Schicht

(a1) einen Rotigotinanteil von mindestens 20 Gew%, bevorzugt mindestens 25 Gew% enthält,

(a2) einen Rotigotingehalt von mindestens 2,0 mg/cm$^2$ bevorzugt 2,8 mg/cm$^2$, besonders bevorzugt mindestens 3,1 mg/cm$^2$ oder mindestens 3,4 mg/cm$^2$ aufweist,

(a3) optional eine die Wirkstofffreisetzung retardierende Menge eines organischen Wachses und/oder einer inneren Phase-Komponente enthält, und

(b) nach dem Auftrag des TTS auf der Haut des Patienten Rotigotin über einen Zeitraum von mindestens 5 Tagen, bevorzugt mindestens 7 Tagen, in einer steady-state Fluxrate von 100-500 μg/Stunde, bevorzugt 200-300 μg/ Stunde durch die Haut abgegeben wird.

Ein weiterer Gegenstand der Erfindung ist ein Rotigotin-haltiges TTS, vorzugsweise ein Rotigotin-haltiges Heißschmelz TTS und besonders bevorzugt ein Rotigotin-haltiges silikonbasiertes Heißschmelz TTS, dadurch gekennzeichnet, dass

(a) das Rotigotin in der Klebermatrix in einer Menge von mindestens 20 Gew%, bevorzugt mindestens 25 Gew% enthalten ist,

(b) die Klebermatrix einen Rotigotingehalt von mindestens 2,0 mg/cm$^2$, bevorzugt 2,8 mg/cm$^2$, besonders bevorzugt mindestens 3,1 mg/cm$^2$ oder mindestens 3,4 mg/cm$^2$aufweist und

(c) Rotigotin am Patienten über einen Zeitraum von mindestens 5, 6 oder 7 Tagen in einer steady-state Rate von mindestens 100-500 μg/Stunde, bevorzugt 200-300 μg/Stunde und ganz besonders bevorzugt 230-270 μg/Stunde abgegeben wird.

Das zur Messung der Fluxrate durch Humanhaut eingesetzte in-vitro Modell nach Tanojo (J Contr. Release 45 (1997) 41-47) hat sich bei der Standardisierung des lösungsmittel-basierten, rotigotinhaltigen Silikon-TTS als sehr gutes Modell zur Vorhersage der in vivo Fluxrate, die in klinischen Studien bestimmt wurde, erwiesen. Im Gegensatz zu einigen anderen vergleichsweise eingesetzten in-vitro Humanhautmodellen korrelierten die im Modell nach Tanojo bestimmten in-vitro Fluxraten durch Humanhaut ausgezeichnet mit den in klinischen Studien (Phase III) erzielten Fluxraten, Plasmaspiegeln und klinischen Parametern, z.B. dem UPDRS-Score.

Aus den Ergebnissen des in Ausführungsbeispiel 9 beschriebenen Modells kann daher abgeleitet werden, dass das erfindungsgemäße Heißschmelz TTS auch in vivo zur Verabreichung von Rotigotin in therapeutisch relevanter Menge über mehrere Tage geeignet ist.

Bevorzugt wird dabei in der klinischen Praxis die Fluxrate so eingestellt, dass am Patienten ein kontinuierlicher therapeutischer Plasmaspiegel zwischen 0,4 und 2 ng/mL Blut erreicht wird. Hierzu ist ein stündlicher Rotigotin-Flux durch die Haut des Patienten von 100-400 μg, bevorzugt von etwa 200-300 μg Rotigotin (entspricht 10-15 μg/cm$^2$/h für ein 20 cm$^2$ TTS), besonders bevorzugt 230-270 μg und ganz besonders bevorzugt etwa 250 μg notwendig. Abweichun-gen der Standard-Dosierung können sich insbesondere auf Grund der Konstitution des Patienten ergeben. Wie Abbildung 6b zeigt, ist eine solche Fluxrate mit den erfindungsgemäßen TTS über einen Zeitraum von 7 Tagen erreichbar.

Damit wurde erstmals ein TTS zur kontinuierlichen transdermalen Verabreichung von Rotigotin zur Verfügung gestellt, daß nach dem Auftrag auf menschliche Haut Rotigotin über einen Zeitraum von mindestens 5, 6 oder 7 Tagen eine mittlere Plasmakonzentration von 0.4 - 2 ng/ml Rotigotin induziert.

Ein anderer Aspekt der vorliegenden Erfindung ist ein TTS, vorzugsweise ein Heißschmelz-TTS, besonders bevorzugt ein silikonbasiertes Heißschmelz-TTS, das zur kontinuierlichen Verabreichung von Rotigotin am Menschen über einen Zeitraum von mindestens 5, 6 oder 7 Tagen geeignet ist, wobei über mindestens 80%, bevorzugt über mindestens 90% und besonders bevorzugt über mindestens 95% des gewählten Zeitraums im Kreislauf des Patienten ein Plasmaspiegel von zwischen 0,4 und 2 ng Rotigotin pro mL Blut eingestellt wird.

Die vorstehenden Ausführungen gelten auch für erfindungsgemäße TTS, die Rotigotin-Prodrugs, z.B. Ester oder Carbamate, enthalten. Nach Verabreichung der entsprechenden Mengen Prodrug wird das Rotigotin durch Spaltung in der Haut und/oder im Blut aus dem Prodrug freigesetzt.

Weitere Bestandteile, die prinzipiell in der Kleberschicht enthalten sein können, wie z.B. Antioxidantien, Stabilisatoren, Tackifier, Konservierungsmitel oder Penetrationsförderer sind dem Fachmann bekannt. Ob die Hinzufügung solcher Bestandteile zu den wesentlichen Bestandteilen der Erfindung, wie in den Ansprüchen definiert, im Einzelfall nützlich ist, kann durch Routine-Versuche ermittelt werden. Diese Ausführungsformen werden daher explizit zum Gegenstand der Erfindung gemacht.

In einer bevorzugten Ausführungsform der Erfindung enthalten die erfindungsgemäßen Heißschmelz-TTS keine Penetrationsförderer.

Eine Ausführungsform der Erfindung ist daher ein Heißschmelz-TTS, umfassend eine Klebermatrix, die enthält:

(a) 50-99 Gew% eines heißschmelzfähigen Haftklebers,

(b) 1-40 Gew%, bevorzugt 5-30 Gew%, besonders bevorzugt 9-30 Gew% und ganz besonders bevorzugt 15-25 Gew% oder 20-30 Gew% Rotigotin,

(c) 0-40 Gew%, bevorzugt 2-25 Gew%, besonders bevorzugt 5-25 Gew% einer inneren Phase-Komponente, die bevorzugt ausgewählt ist aus der Gruppe Polysaccharide, substituierte Polysaccharide, Polyethylenoxide, Polyvinylace, Polyvinylpyrrolidone mit oder ohne Weichmacher, Polyethylenglycol, Polypropylenglycol, Acrylate, Copolymere aus Polyvinylpyrrolidon und (Poly)vinylacetat, Copolymere von Ethylen und Vinylacetat sowie Polyvinylalkohole mit Weichmacher, z.B. Glycerin,

(d) 0-10 Gew%, bevorzugt 0-5 Gew% und besonders bevorzugt 0-3 Gew% weitere Hilfsstoffe, z.B. Tackifier, Antioxidantien, Stabilisatoren, Penetrationsförderer,

wobei der heißschmelzfähige Haftkleber (a) bevorzugt eine Mischung ist aus

(i) 70-99 Gew% eines amin-resistenten Silikonklebers,

(ii) 1-30 Gew% eines geeigneten Weichmachers, insbesondere eines Wachses, besonders bevorzugt eines organischen Wachses und ganz besonders bevorzugt Ozokerit oder Ceresin

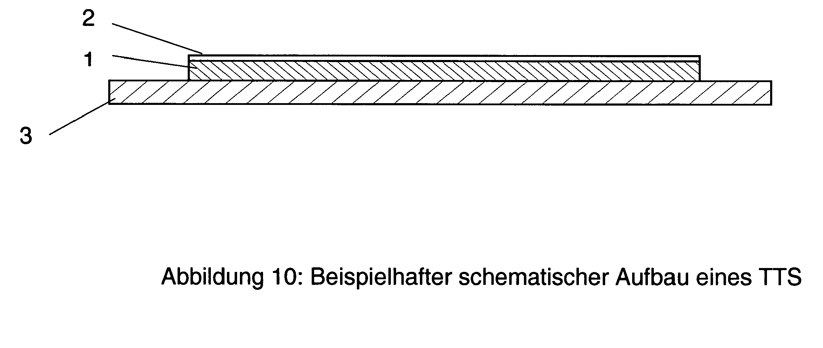

Das Heißschmelz-TTS kann ausschließlich aus der Klebermatrix bestehen, enthält neben der Rotigotin-haltigen Klebermatrix bevorzugt aber als weitere Bestandteile eine Wirkstoffundurchlässige und für die Bestandteile der Klebermatrix inerte Rückschicht (2) sowie eine die Klebermatrix (1) abdeckende, vor Gebrauch zu entfernende Schutzfolie (3) (siehe Abbildung 10). Weitere Varianten des TTS-Aufbaus, die z.B. zusätzlich eine den Wirkstoff-flux steuernde Membran und/oder eine zusätzliche Klebstofffolie ("Overtape") enthalten, sind dem Fachmann bekannt. Besonders bevorzugt wird der in Abbildung 10 dargestellte "monolithische" TTS-Aufbau.

Rotigotin ist ein Dopamin-Agonist. Das erfindungsgemäße TTS ist somit insbesondere zur Behandlung von Erkrankungen geeignet, die mit einem gestörten Dopamin-Stoffwechsel einhergehen, ganz besonders zur Behandlung von Morbus Parkinson oder Restless Leg.

Ein Gegenstand der Erfindung ist daher eine Methode zur Behandlung einer Dopamin-Stoffwechselerkrankung, insbesondere Morbus Parkinson oder Restless Leg Syndrom,

dadurch gekennzeichnet, dass auf die Haut eines Patienten ein erfindungsgemäßes Rotigotin-haltiges Heißschmelz-TTS aufgetragen wird.

Ein weiterer Gegenstand der Erfindung ist eine Verkaufspackung enthaltend ein oder mehrere erfindungsgemäße Rotigotin-haltige Heißschmelz TTS sowie Anleitungen zu deren Verwendung.

Aus dem Stand der Technik sind bisher nur Verfahren zur Herstellung von Rotigotinhaltigen TTS bekannt, in denen die Rotigotin-haltige Klebermatrix durch das Entfernen von Lösungsmitteln aus einer lösungsmittelhaltigen Silikon- oder Acrylat-basierten Dispersion erfolgt. Mit der vorliegenden Erfindung wurde erstmals ein lösungsmittelfreies Heißschmelzverfahren zur Herstellung eines Rotigotin-haltigen TTS zur Verfügung gestellt.

Ein Aspekt der vorliegenden Erfindung ist daher ein Verfahren zur Herstellung eines TTS umfassend eine Rotigotin als Wirkstoff enthaltende Klebermatrix, dadurch gekennzeichnet, dass die Bestandteile der Klebermatrix vor dem Laminieren auf Folie bei Temperaturen zwischen 70 und 200°C, bevorzugt zwischen 100 und 200°C und besonders bevorzugt zwischen 120 und 160°C, lösungsmittelfrei geschmolzen und homogenisiert werden. Die ganz besonders bevorzugte Arbeitstemperatur im Extruder liegt zwischen 130 und 150°C.

Es zeigte sich dabei überraschend, dass das Rotigotin nach Schmelzen in verschiedenen Matrices auch ohne den Zusatz von Stabilisierungsmitteln oder Antioxidantien stabil bleibt. HPLC-Messungen mit UV-detektion bei 220 nm und 272 nm zeigten, dass auch ohne Zugabe von Antioxidantien der Reinheitsgrad des Wirkstoffs regelmäßig über 98% und im allgemeinen über 99% lag (Tabellen 2-4; Ausführungsbeispiele 4,6,7).

In einem Aspekt betrifft die Erfindung daher die Verwendung von Rotigotin bei der Herstellung eines TTS, dadurch gekennzeichnet, daß das Rotigotin im Heißschmelzverfahren in die Kleberschicht des TTS eingebracht wird.

Das Rotigotin kann grundsätzlich in vorgeschmolzener Form in die Matrix eingebracht werden oder aber in fester Form in die heiße Matrixschmelze eindosiert und dabei aufgeschmolzen werden.

In einer bevorzugten Ausführungsform findet das Schmelzen des Rotigotins bei Temperaturen zwischen 100 und 200°C, bevorzugt zwischen 120 und 160°C und besonders bevorzugt zwischen 130°C und 150°C durch die Eindosierung festen Rotigotins in die Matrixschmelze statt und kann, optional, in Abwesenheit zusätzlicher Stabilisatoren oder Antioxidantien durchgeführt werden.

In einer besonders bevorzugten Ausführungsform wird das Rotigotin geschmolzen, in dem es in fester Form in die heiße Matrixschmelze eindosiert wird, wobei die Rotigotin-haltige Matrixschmelze nach kurzem Homogenisieren auf Folie kalandriert und abgekühlt wird. Bevorzugt wird das Rotigotin dabei nur für höchstens 5 Minuten, besonders bevorzugt weniger als 4, 3, 2 oder sogar weniger als 1 Minute einer Temperatur von 100°C bis 200°C, bevorzugt 120-160°C und besonders bevorzugt 130-150°C ausgesetzt.

Ein weiterer Aspekt der Erfindung ist daher die Verwendung von Rotigotin zur Herstellung eines TTS im Heißschmelzverfahren bei Temperaturen zwischen 120 und 160°C und besonders bevorzugt bei 130°C bis 150°C, wobei das Heißschmelzverfahren zur Herstellung einer Klebermatrix mit einem Rotigotin-Reinheitsgehalt von mindestens 98%, bevorzugt 99%, gemessen bei 220 und 272 nm, führt.

In einer anderen Ausführungsart der Erfindung wird die Kleberschicht des TTS bei sehr niedrigen Temperaturen von 70-75°C, das heißt knapp unterhalb des Schmelzpunktes von Rotigotin, geschmolzen. In dieser Ausführungsform liegt das Rotigotin daher zunächst in fester Form in der Matrix vor. Für diese Ausführungsart müssen heißschmelzfähige Haftkleber verwendet werden, die zur Verarbeitung bei 70°C geeignet sind, wobei andererseits die dynamische Viskosität der Haftklebermischung nicht zu niedrig eingestellt sein darf, um den Kaltfluss der Kleberschicht auf der Haut zu vermeiden. Daher müssen bei diesem Verfahren recht hohe Scherkräfte angelegt werden.

Ein Aspekt der Erfindung ist daher die Herstellung eines TTS im Heißschmelzverfahren, wobei die Kleberschicht bei Temperaturen unterhalb des Schmelzpunktes von Rotigotin, das heißt unter ca. 75°C geschmolzen wird und das Rotigotin in fester Form in die Schmelze eindosiert wird.

Zur industriellen Produktion des TTS wird die Kleberschicht bevorzugt in einem Extruder hergestellt. Die einzelnen Bestandteile der Kleberschicht können dabei separat oder aber vorgemischt in die verschiedenen Zuführungen des Extruders, z.B. eines Zweischneckenextruders eingeführt werden. Die entstehende Mischung wird im Extruder unter kontrollierter Erwärmung gemischt und kann kontinuierlich prozessiert und schließlich laminiert werden.

Da der heißschmelzfähige Haftkleber bei Raumtemperatur in fester Konsistenz vorliegt, ist ein Vorschmelzen erforderlich. Dies kann beispielsweise durch ein Schmelz-Dosierungssystem bewerkstelligt werden, das aus einem Container mit kontrollierbarer Erwärmung besteht, in dem der heißschmelzfähige Haftkleber, beispielsweise der heißschmelzfähige Silikonhaftkleber, bei Temperaturen zwischen 70°C und 200°C, bevorzugt zwischen 100°C und 170°C und besonders bevorzugt zwischen 120°C und 160°C ganz besonders bevorzugt zwischen 130 und 150°C vorgeschmolzen wird. Das Schmelzdosierungssystem kann kontinuierlich nachbeladen werden und somit problemlos in das kontinuierliche System integriert werden. Das Dosierungssystem kann zur volumetrischen oder gravimetrischen Dosierung geeignet sein.

Rotigotin ist in hydrophoben Klebern, wie z.B. in Silikonen, nur in Spuren löslich und muss daher dispergiert werden. Die Viskosität des geschmolzenen Rotigotins ist sehr niedrig, so dass während des Prozesses beträchtliche Viskositätsunterschiede zwischen dem Haftkleber und dem Wirkstoff bestehen können. Um eine optimale Verteilung des Wirkstoffs in der Klebermatrix zu erreichen, können daher optional statische Mischer in den Extruderprozeß integriert werden, die eine noch homogenere Durchmischung der Klebermatrix gewährleisten. Geeignete statische Mischer können beispielsweise von der Firma Sulzer Chemtech GmbH bezogen werden. Auf diese Weise gelang es, die Tröpfchengröße der Wirkstoffpartikel bzw. der inneren-Phase Domänen auf eine durchschnittliche Größe unter 20 $\mu$m zu senken, wie mikroskopische Untersuchungen der Klebematrix zeigten.

Dies hat mehrere Vorteile:

Zum einen wird dadurch die Entstehung größerer Wirkstoffreservoirs in der Matrix verhindert, die zu ungleichmäßigem Wirkstoffflux, zur Beeinträchtigung der Adhäsions/Kohäsionsbalance der Klebermatrix oder zur Rekristallisierung des Wirkstoffs führen können.

Zum anderen wird eine Anreicherung von Wirkstoff an der Grenzfläche Klebermatrix/Haut verhindert, was zur Hautreizung und/oder zur Wirkstoffprotonierung mit der Folge verminderter Fluxrate durch Rückdiffusion der protonierten Base führen kann.

Die maximale Größe der Mikroreservoire sollte daher 80%, bevorzugt 60% der Dicke der Klebermatrix, besonders bevorzugt 50% der Dicke der Klebermatrix nicht überschreiten. Die mittlere Größe der Mikroreservoire liegt bevorzugt im Bereich bis 40% und besonders bevorzugt bis 30% der Matrixdicke.

Bezogen auf eine beispielhafte Matrixdicke von 50 $\mu$m liegt die innere Phase in der Klebermatrix daher bevorzugt in Form von Tröpfchen einer mittleren Größe von bis zu 20 $\mu$m, bevorzugt bis maximal 15 $\mu$m vor.

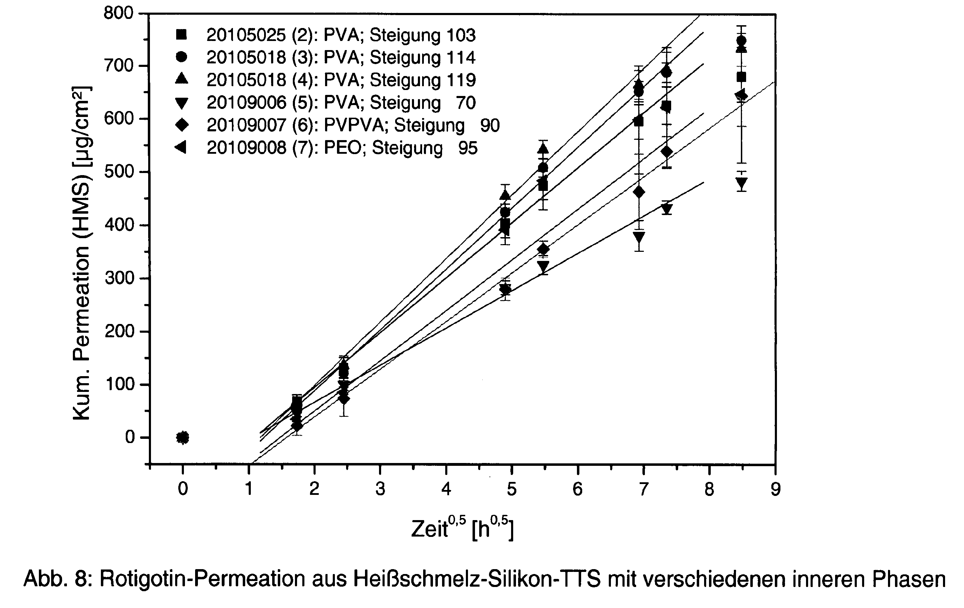

Abbildung 8 zeigt die in-vitro Permeation von Rotigotin durch Mäusehaut aus verschiedenen silikonbasierten Heißschmelz-TTS, die per Schmelzextrusion im Extruder hergestellt wurden, wobei verschiedene innere Phase-Komponenten eingesetzt wurden.

Neben den silikonbasierten Haftklebersystemen kommen zur Verwendung in den erfindungsgemäßen Rotigotin-haltigen Heißschmelz-TTS grundsätzlich auch andere heißschmelzfähige Haftkleber in Betracht.

Heißschmelzfähige Haftkleber sind aus dem Stand der Technik bekannt. Beispielsweise können Styrol-Block-Copolymer-basierte heißschmelzfähige Kleber ("SXS-Haftkleber") verwendet werden, die auf Polymeren mit nicht-elastomeren Styrolblöcken an den Enden und elastomeren Blöcken in der Mitte beruhen. Die elastomeren Blöcke können beispielsweise aus Polyethylenbutylen, Polyethylenpropylen, Polybutadien oder Polyisopropen bestehen.

Solche Kleber werden beispielsweise in US 5,559,165 und US 5,527,536 beschrieben und zeichnen sich aus durch gute Klebeeigenschaften, einfache Herstellung und Verarbeitung und gute Hautverträglichkeit. SXS-Haftkleber können sowohl kommerziell bezogen werden (z.B. als Duro Tak 378-3500 bei National Starch & Chemical), als auch mit einem Heißschmelz Extrusions-Equipment selber bei der Produktion der wirkstoffhaltigen Pflaster hergestellt werden. Dazu werden entsprechende Mengen (wenigstens folgender Bestandteile) eines Styrol-Block-Copolymers (z.B. Shell Kraton GX1657 oder Kraton D-1107CU) mit einem Harz (z.B. Keyser Mackay Regalite R1090 oder Regalite R1010 oder Regalite R1100) und einem Öl (z.B. Shell Ondina 933 oder Ondina 941) aus den einzelnen Dosierstationen in den Extruder dosiert, dort vermischt und aufgeschmolzen. Im letzten Schritt wird in den so hergestellten Haftkleber der Wirkstoff in den Extruder eindosiert und die Masse auf Folien laminiert. Typische exemplarische Gewichtsanteile Polymer : Harz : Öl sind z.B. 100:120:20 oder 100:200:50. Durch Variation dieser Mengenanteile können die Eigenschaften des SXS-Haftklebers jeweils an die gewünschten Eigenschaften des TTS (Klebkraft, minimaler Kaltfluß, Klebezeitdauer, Freisetzungsprofil des Wirkstoffes, etc.) angepaßt werden.

Wegen der oxidativen Wirkung der SXS-Kleber werden SXS-basierten Klebermatrices bevorzugt Antioxidantien zugesetzt. Ein Beispiel für ein kommerziell erhältliches, geeignetes Antioxidans ist Irganox[R] (CIBA).

Ein anderes Beispiel sind heißschmelzfähige Haftkleber, die auf Ethylenvinylacetat-Copolymeren beruhen ("EVA-Haftkleber"). Solche EVA-Kleber werden beispielsweise beschrieben in US 4,144,317. EVA-Kleber zeichnen sich aus durch gute Klebeeigenschaften, einfache Herstellung und Verarbeitung und gute Hautverträglichkeit. EVA-Kleber können bezogen werden, z.B. bei Beardow Adams (13/BA).

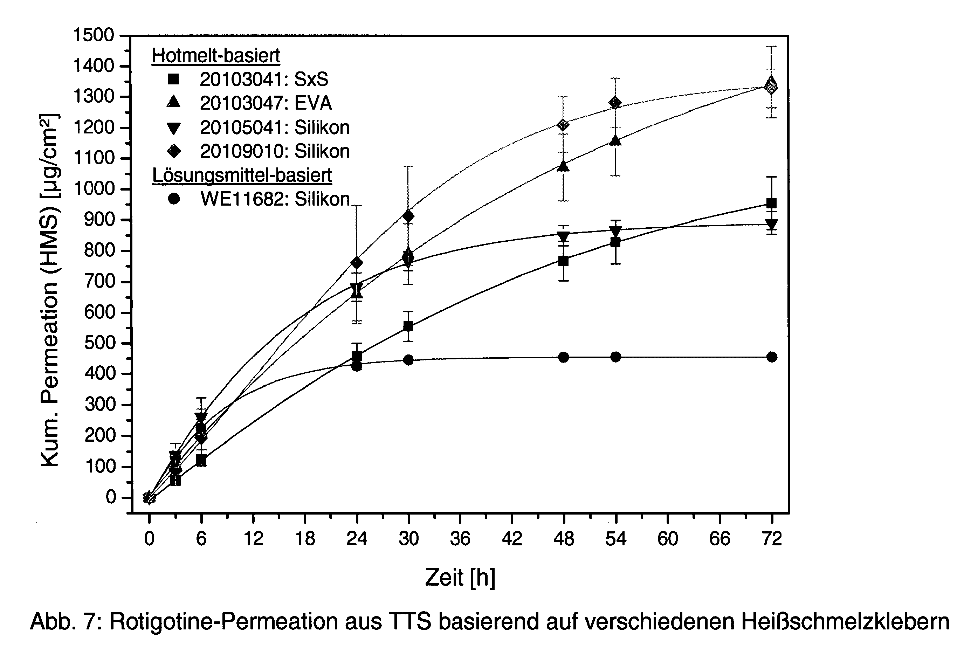

Sowohl mit heißschmelzfähigen Haftklebern vom SXS-Typ als auch mit heißschmelzfähigen Haftklebern vom EVA-Typ konnten Rotigotin-haltige TTS hergestellt werden, die Heißschmelz-Klebematrices enthielten und die Rotigotin in relevanter Menge freisetzten (Abb. 7).

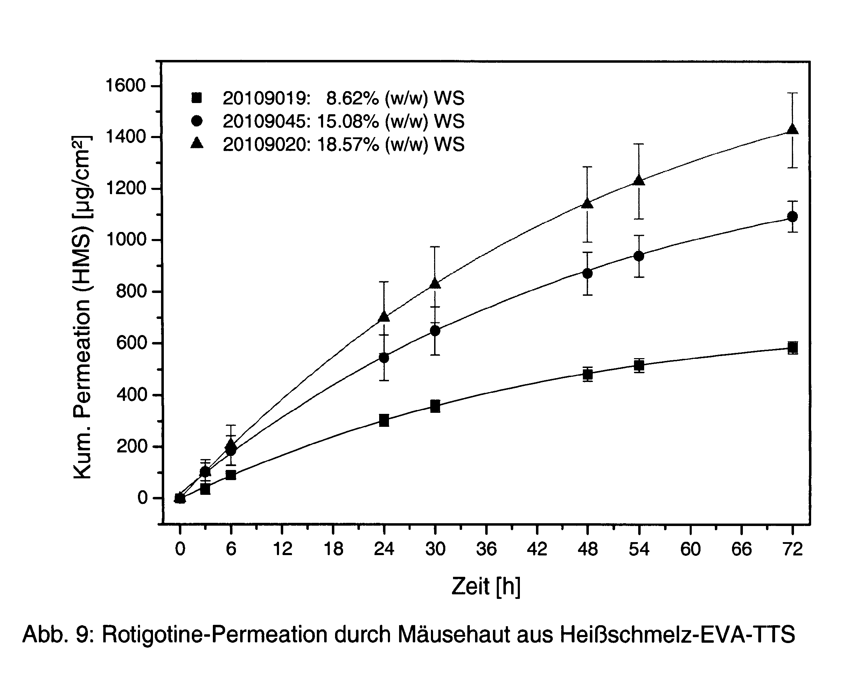

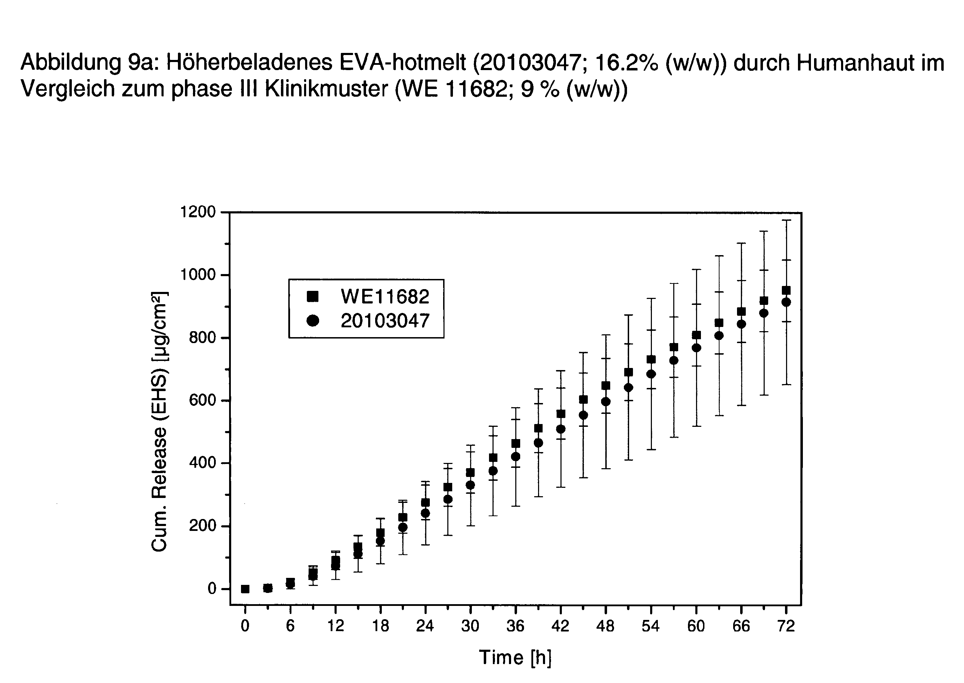

Abbildungen 9 und 9a zeigen die in-vitro Permeation von Rotigotin durch Mäusehaut bzw. durch Humanhaut aus EVA-basierten Heißschmelz-TTS mit unterschiedlichem Rotigotin-Gehalt, die per Schmelzextrusion im Extruder hergestellt wurden.

Ein Gegenstand der Erfindung ist daher ein transdermales therapeutisches System (TTS) enthaltend eine Rotigotin-haltige Klebermatrix, dadurch gekennzeichnet, dass die Klebermatrix einen heißschmelzfähigen Haftkleber enthält, in dem der Wirkstoff Rotigotin ((-)-5,6,7,8-Tetrahydro-6-(propyl[2-(2-thienyl)ethyl)amino]-1-naphtol) dispergiert, teilweise oder vollständig gelöst ist, wobei der heißschmelzfähige Haftkkleber vom SXS-Typ oder vom EVA-Typ ist.

Eine Ausführungsform der Erfindung ist daher ein Heißschmelz-TTS, umfassend eine Klebermatrix, die enthält:

(a) 50-99 Gew% eines heißschmelzfähigen Haftklebers,
(b) 1-40 Gew%, bevorzugt 5-30 Gew%, besonders bevorzugt 9-30 Gew% und ganz besonders bevorzugt 15-25 Gew% Rotigotin oder ein Prodrug von Rotigotin,
(c) 0-40 Gew%, bevorzugt 2-25 Gew%, besonders bevorzugt 5-25 Gew% einer inneren Phase-Komponente, die bevorzugt ausgewählt ist aus der Gruppe Polysaccharide, substituierte Polysaccharide, Polyethylenoxide, Polyvinylace, Polyvinylpyrrolidone mit oder ohne Weichmacher, Polyethylenglycol, Polypropylenglycol, Acrylate, Copolymere aus Polyvinylpyrrolidon und (Poly)vinylacetat, Copolymere von Ethylen und Vinylacetat sowie Polyvinylalkohole mit Weichmacher, z.B. Glycerin,
(d) 0-10 Gew%, bevorzugt 0-5 Gew% und besonders bevorzugt 0-3 Gew% weitere Hilfsstoffe, z.B. Tackifier, Antioxidantien, Stabilisatoren, Penetrationsförderer

wobei der heißschmelzfähige Haftkleber aus (a) bevorzugt ausgewählt ist aus

(a1) einem EVA-Haftkleber,
(a2) einem SXS-Haftkleber oder
(a3) einem Gemisch aus

(i) 70-99 Gew% eines amin-resistenten Silikonklebers,
(ii) 1-30 Gew%, bevorzugt 3-15 Gew% und ganz besonders bevorzugt 4-10 Gew% eines geeigneten Weichmachers, bevorzugt ein organisches Wachs und besonders bevorzugt Ceresin oder Ozokerit,

wobei dem EVA-Haftkleber (a1) und dem SXS-Haftkleber (a2) optional Weichmacher zugesetzt sein können und, wenn ein SXS-Haftkleber zugegen ist, ein Antioxidans zugesetzt wird.

Soll ein per se heißschmelzfähiger Haftkleber, beispielsweise vom SXS-Typ oder vom EVA-Typ, auf bestimmte Verarbeitungserfordernisse eingestellt werden, können auch hier optional weitere Rezepturbestandteile, z.B. weitere Weichmacher, Tackifier, Antioxidantien, amphiphile Polymere etc. zugesetzt werden.

Im Vergleich der Freisetzung von Rotigotin aus verschiedenen Heißschmelzklebern zeigte sich, dass die Effizienz des TTS mit dem silikonbasierten Heißschmelzkleber am größten ist. Ein Absinken der Rotigotin-Freisetzung auf ein therapeutisch nicht mehr effektives Level tritt aus dem SXS-basierten und EVA-basierten Heißschmelz-TTS zu einem Zeitpunkt auf, zu dem sich noch ca. 30 Gew% des Rotigotins in der Klebermatrix befinden. Demgegenüber konnte das silikonbasierte Heißschmelz-TTS nahezu vollständig ausgeschöpft werden.

Daher wird ein Rotigotin-haltiges Heißschmelz-TTS auf Basis eines silikonbasierten Haftschmelzklebers, wie weiter oben beschrieben, ganz besonders bevorzugt.

Die ursprünglich eingereichten Ansprüche der Internationalen Anmeldung mit der originalen Nummerierung lauten wie folgt:

1. Transdermales Therapeutisches System (TTS) umfassend eine den Wirkstoff Rotigotin ((-)-5,6,7,8-Tetrahydro-6-[propyl[2-(2-thienyl)ethyl)amino]-1-naphtol) enthaltende wirkstoffhaltige Klebermatrix, dadurch gekennzeichnet, dass die Klebermatrix einen heißschmelzfähigen Haftkleber enthält.

2. TTS nach Anspruch 1, wobei das Rotigotin im heißschmelzfähigen Haftkleber dispergiert, teilweise oder vollständig gelöst ist.

3. TTS nach einem der vorhergehenden Ansprüche, wobei die Rotigotin-haltige Klebermatrix hergestellt wurde, indem das Rotigotin bei einer Temperatur zwischen 70°C und 200 °C in die lösungsmittelfreie Schmelze der Klebermatrix dosiert wurde.

4. TTS nach einem der vorhergehenden Ansprüche, wobei der heißschmelzfähige Haftkleber aus einer Mischung eines amin-resistenten Silikonhaftklebers mit mindestens einem geeigneten Weichmacher besteht.

5. TTS nach Anspruch 4, wobei der Weichmacher ein organisches Wachs ist.

6. TTS nach einem der Ansprüche 4-5, wobei der Weichmacher Ceresin oder Ozokerit ist.

7. TTS nach einem der vorhergehenden Ansprüche, wobei das Rotigotinin in einem Anteil von 4-40 Gew% in der Kleberschicht vorliegt.

8. TTS nach einem der vorhergehenden Ansprüche, wobei das Rotigotin in einem Anteil von 9-30 Gew% in der Kleberschicht vorliegt.

9. TTS nach einem der Ansprüche 1-7, wobei das Rotigotin in einem Anteil von 20-40 Gew% in der Kleberschicht vorliegt.

10. TTS nach einem der vorhergehenden Ansprüche, wobei das Rotigotinin als Wirkstoffbase vorliegt.

11. TTS nach einem der vorhergehenden Ansprüche, wobei die wirkstoffhaltige Klebermatrix ferner eine innere Phase-Komponente enthält, die ausgewählt ist aus der Gruppe

    (a) hydrophiler oder amphiphiler Polymere,
    (b) hydrophiler oder amphiphiler Copolymere,
    (c) Mischungen aus (a) und/oder (b) mit pharmazeutisch akzeptablen Weichmachern,
    (d) Kondensate aus Glycerin und Fettsäuren oder Polyolen,
    (e) geeignete Mischungen aus den Bestandteilen (a)-(d).

12. TTS nach Anspruch 11, wobei die innere Phase-Komponente ausgewählt ist aus der Gruppe Polysaccharide, substituierte Polysaccharide, Polyethylenoxide, Polyvinylacetate, Polyvinylpyrrolidone, Copolymere aus Polyvinylpyrrolidon und (Po-ly)vinylacetat, Polyethylenglycol, Polypropylenglycol, Copolymere von Ethylen und Vinylacetat, Glycerin-Fettsäureester sowie Mischungen aus Polyvinylalkohol mit Glycerin.

13. TTS nach einem der Ansprüche 1-3, dadurch gekennzeichnet, dass die Klebermatrix umfasst

    (a) 50-99 Gew% eines heißschmelzfähigen Haftklebers,
    (b) 4-40 Gew% Rotigotin,
    (c) 0-40 Gew% innere Phase-Komponente,
    (d) 0-10 Gew% weitere Hilfsstoffe.

14. TTS nach einem der Ansprüche 1-3, wobei der heißschmelzfähige Haftkleber ausgewählt ist aus

    (a1) einem EVA-Haftkleber,

(a2) einem SxS-Haftkleber oder

(a3) einem Gemisch aus

(i) 70-99 Gew% eines amin-resistenten Silikonklebers,

(ii) 1-30 Gew% eines geeigneten Weichmachers.

15. TTS zur kontinuierlichen transdermalen Verabreichung von Rotigotin, dadurch gekennzeichnet, dass das TTS nach dem Auftrag auf menschliche Haut über einen Zeitraum von mindestens 5 Tagen beim Patienten eine mittlere Plasmakonzentration von 0.4 - 2 ng/ml Rotigotin induziert.

16. TTS nach Anspruch 15 wobei das TTS über einen Zeitraum von mindestens 5 Tagen beim Patienten eine mittlere Plasmakonzentration von 0.4 - 2 ng/ml Rotigotin induziert.

17. TTS zur kontinuierlichen transdermalen Verabreichung von Rotigotin, dadurch gekennzeichnet, dass Rotigotin über einen Zeitraum von mindestens 5 Tagen in einer steady-state Fluxrate von 200-300 $\mu$g pro Stunde durch die Haut transportiert wird.

18. TTS nach einem der Ansprüche 15-17, wobei das TTS eine Rotigotin-haltige Schicht enthält, die dadurch gekennzeichnet ist, dass die wirkstoffhaltige Schicht

(a) einen Rotigotinanteil von mindestens 20 Gew% enthält,

(b) einen Rotigotingehalt von mindestens 2,0 mg/cm$^2$ aufweist und

(c) optional eine die Wirkstofffreisetzung retardierende Menge eines organischen Wachses und/oder einer inneren Phase-Komponente enthält.

19. Verfahren zur Herstellung eines TTS, umfassend eine Rotigotin als Wirkstoff enthaltende Klebermatrix, dadurch gekennzeichnet, dass die Bestandteile der Klebermatrix vor dem Laminieren bei Temperaturen zwischen 70°C und 200°C lösungsmittelfrei geschmolzen und homogenisiert werden.

20. Verfahren nach Anspruch 19, dadurch gekennzeichnet, dass das Schmelzen und Homogenisieren der Bestandteile der Klebermatrix im Extruder durchgeführt wird.

21. Verwendung von Rotigotin bei der Herstellung eines TTS im Heißschmelzverfahren, dadurch gekennzeichnet, dass das Rotigotin bei Temperaturen zwischen 70°C und 200°C in die lösungsmittelfrei vorgeschmolzene Klebermatrix des TTS eingebracht wird.

22. Verfahren oder Verwendung nach einem der vorhergehenden Ansprüche, wobei das Heißschmelzverfahren bei Temperaturen zwischen 120°C und 160°C stattfindet.

23. Verfahren oder Verwendung nach einem der vorhergehenden Ansprüche, wobei das Rotigotin in fester Form in die Schmelze der Klebermatrix eingebracht wird.

24. Verfahren oder Verwendung nach einem der vorhergehenden Ansprüche, wobei das Rotigotin in der durch Heißschmelzverfahren hergestellten Klebermatrix in einer Reinheit von mindestens 98%, gemessen per HPLC bei 220 nm und 272 nm, vorliegt.

25. TTS, Verfahren oder Verwendung nach einem der vorhergehenden Ansprüche, wobei statt Rotigotin ein Prodrug von Rotigotin verwendet wird bzw. zugegen ist.

26. TTS, Verfahren oder Verwendung nach Anspruch 25, wobei das Rotigotin-Prodrug ein Ester oder Carbamat von Rotigotin ist.

**Experimenteller Teil:**

Vergleichsbeispiel: Lösunasmittel-basiertes Silikon-TTS

1.8 g Rotigotin (freie Base) werden in 2.4 g Ethanol gelöst und zu 0.4 g Kollidon 90F (gelöst in 1g Ethanol) gegeben. Diese Mischung wird zu einer 74%igen Lösung von Silikonpolymeren (8.9 g BioPSA 7-4201 + 8.9 g BIO-PSA 7-4301 [Dow Corning]) in Heptan gegeben. Nach Zugabe von 2.65 g Petrolether wird die Mischung für 1 Stunde bei 700 UpM gerührt, um eine homogene Dispersion zu erhalten. Nach Laminierung auf Polyester wurde bei 50°C getrocknet. Das Pflastergewicht betrug schließlich 50 g/cm$^2$.

1. Beispiel: Herstellung eines silikonbasierten Heißschmelz-TTS mit 15% Rotigotin im Labormaßstab

(a) Silikon-Heißschmelzhaftkleber

Es wurden silikonbasierte Heißschmelzkleber verwendet, die den Silikonkleber Bio-PSA 7-4300 (Dow Corning, Michigan) in Mischung mit den Weichmachern Ozokerit oder Ceresin in Konzentrationen von 5%, 10% oder 15% des Gesamtgewichts der Haftklebermischung enthielten (bezogen bei Dow Corning).

(b) Herstellung des TTS

8,5 g einer silikonbasierten Haftklebermischung, wie in (a) beschrieben, wurde für etwa 20 Minuten auf 160°C erhitzt, bis eine homogene Schmelze entstand. 1.5 g Rotigotin (freie Base) wurde zugesetzt und das Gemisch für weitere 5 Minuten bei 160°C gehalten. Die Mischung wurde dann manuell homogenisiert und auf eine vorgewärme Folie (120°C, Spaltweite 250 μm) laminiert. Es wurden 5cm$^2$ Stücke ausgeschnitten.

2. Beispiel: Herstellung eines silikonbasierten Heißschmelz-TTS mit innerer Phase

Die Herstellung erfolgte entsprechend Beispiel 1, wobei gemeinsam mit dem Rotigotin noch 0.5 g innere-Phase Komponente zugesetzt wurde.

3. Beispiel: Herstellung eines silikonbasierten Heißschmelz-TTS im Labormaßstab unter Variation der Parameter

Die TTS wurden grundsätzlich hergestellt wie in Beispielen 1 und 2 beschrieben. Dabei wurden die verschiedenen Parameter wie Wachsart, Wachsgehalt, Konzentration der innere-Phase-Komponente, Wirstoffgehalt, Pflasterdichte wie folgt variiert:

**Tabelle 1: Silikon-basierte Heißschmelz-TTS**

| Lot No (Ch.B.) | Ceresin-Gehalt [% w/w] | Ozokerit-Gehalt [% w/w] | Innere Phase-Typ/Gehalt [% w/w] | theoret. Rotigotin Gehalt [% w/w] | Gemessener Rotigotin-Gehalt (n=5) [% w/w] | Gewicht der Klebermatrix (n=10) [g/m$^2$] |
|---|---|---|---|---|---|---|
| 20011031 | 15 | - | PVP/10 | 9 | 8.51 | 108 |
| 20011032 | 15 | - | PVP/2 | 9 | 9.23 | 83 |
| 20011035 | 15 | - | PVP/2 | 15 | 15.81 | 66 |
| 20011036 | 15 | - | PVP/10 | 15 | 15.56 | 100 |
| 20012038 | 15 | - | PVP/2 | 9 | n.d. | 123 |
| 20012040 | 15 | - | PVP/2 | 15 | n.d. | 118 |
| 20012042 | 15 | - | PVP/2 | 25 | n.d. | 114 |
| 20103042 | 15 | - | 0 | 15 | 15.25 | 57 |
| 20103043 | 15 | - | PVP/25 | 15 | 14.04 | 127 |
| 20105038 | 15 | - | 0 | 9 | 8.75 | 91 |
| 20105039 | 15 | - | PVP/2 | 9 | 9.07 | 88 |
| 20105040 | 15 | - | PVP/10 | 9 | 9.14 | 91 |
| 20105041 | 5 | - | 0 | 9 | 8.08 | 106 |
| 20105043 | - | 5 | 0 | 9 | 8.03 | 105 |
| 20105044 | 15 | - | 0 | 15 | 14.50 | 78 |

Tabelle fortgesetzt

| Lot No (Ch.B.) | Ceresin-Gehalt [% w/w] | Ozokerit-Gehalt [% w/w] | Innere Phase-Typ/Gehalt [% w/w] | theoret. Rotigotin Gehalt [% w/w] | Gemessener Rotigotin-Gehalt (n=5) [% w/w] | Gewicht der Klebermatrix (n=10) [g/m²] |
|---|---|---|---|---|---|---|
| 20105045 | 15 | - | 0 | 25 | 25.20 | 77 |
| 20106016 | - | 15 | 0 | 9 | 8.12 | 88 |
| 20107040 | - | 5 | 0 | 15 | 13.71 | 99 |
| 20107041 | - | 5 | 0 | 25 | 24.71 | 84 |
| 20109009 | - | 15 | 0 | 15 | 13.28 | 89 |
| 20109010 | 5 | - | 0 | 15 | 14.09 | 107 |
| 20111059 | - | 5 | 0 | 25 | 23.95 | 54 |
| 20111058 | - | 5 | 0 | 15 | 14.57 | 54 |
| 20111057 | - | 5 | 0 | 9 | 8.64 | 56 |
| 20109043 | - | 5 | 0 | 25 | 22.69 | 117 |
| 20105044 | - | 15 | 0 | 15 | 14.49 | 57 |
| 20103043 | - | 15 | 0 | 15 | 14.04 | 78 |
| WE11682 *) | - | - | PVP/2 | 9 | 8.83 | 50 |
| 20107011*) | - | - | PVP/2 | 9 | 9.90 | 110 |
| *Lösungsmittel-basiertes Vergleichsbeispiel; PVP=Polyvinylpyrrolidon | | | | | | |

Der Rotigotin-Gehalt und das Gewicht der Klebermatrix wurden wie folgt bestimmt: 10 Pflaster a 5 cm², 10 cm² oder 20 cm² wurden ausgestanzt und einzeln gewogen, dieses Gewicht wurde subtraktiv korrigiert durch das mittlere Gewicht der reinen Folien (gemessen an Stücken identischer Größe, also ebenfalls jew. 5, 10 oder 20 cm²).

4. Beispiel: Herstellung SXS oder EVA-basierter Heißschmelz-TTS im Labormaßstab

8,5 g des SXS-Heißschmelzhaftklebers (Duro-Tak 34-4230; National Starch & Chemical) oder 8,5 g des EVA-Heißschmelzklebers wurden für etwa 20 Minuten bei 160°C erhitzt, bis eine homogene Schmelze erhalten wurde. 1,5 g bzw. 1,65 g Rotigotin-Base wurde dazugegeben und die Mischung wurde manuell homogenisiert. Die Mischung wurde dann auf eine vortemperierte (120°C) Chill-Roll laminiert. Es wurden 5cm² (für Permeationsexperimente) und 20cm² Stücke (zur Bestimmung des Pflastergewichts) ausgeschnitten. Das Matrixgewicht kann der nachfolgenden Tabelle 2 entnommen werden.

**Tabelle 2:**

| Lot No (Ch.B.) | Kleber | Innere Phase / Gehalt [% w/w] | Theoretischer Wirkstoff-Gehalt [% w/w] | Gemessener Wirkstoff Gehalt [% w/w] | Gewicht (n=10) [g/m²] | Reinheit % (220 nm/ 272 nm) |
|---|---|---|---|---|---|---|
| 20103041 | SXS | - | 15 | 14.96 | 85 | 94.9/94.3 |
| 20103048 | EVA | - | 16.2 | 18.24 | 58 | 98.1/99.7 |
| 20103047 | EVA | - | 16.2 | 15.96 | 127 | 98.8/99.9 |

5. Beispiel: Herstellung eines silikonbasierten Heißschmelz-TTS mit 15% Rotiaotin und 5% innerere Phase im Extruder

A. Herstellung einer Vorschmelze der Silikon-Haftklebermischung

Die gewünschte Menge der Silikon-Haftklebermischung, wie in Beispiel 1 beschrieben, wurde in ein Dosierungselement (Meltex GR 12-1, Fa. Melzer) gefüllt, in dem das Gemisch auf 140°C vorgewärmt wurde. Die Mischung wurde dann volumetrisch in den Extruder dosiert.

B. Herstellung der Klebermatrix im Heißschmelzverfahren

Es wurde ein Doppelschnecken-Extruder für einen kleinen bis mittleren Produktionsmaßstab (Fa. Dr. Collin GmbH, 25x24D) sowie ein Doppelschnecken-Extruder für den großtechnischen Maßstab (ZSK25, Fa. Werner Pfleiderer, Stuttgart) verwendet. Prozessbedingungen 5 kg/h, Heizzonen 120-140°C. Zum Laminieren wurde ein Melzer CL200 verwendet.

6. Beispiel: Herstellung eines silikonbasierten Heißschmelz-TTS im Extruder unter Variation der Parameter

Das TTS wurde grundsätzlich wie unter Beispiel 5 beschrieben hergestellt. Dabei wurden die Parameter wie folgt verändert:

Tabelle 3:

| Nr | Lot No (Ch.B.) | Maßstab | Statisches Mischen | CeresinGehalt [% w/w] | Innere Phase Typ/ Gehalt [% w/w] | Theoret. Rotigotin Gehalt [% w/w] | Gemessener RotigotinGehalt (n=5) [% w/w] | Matrix Gewicht (n=10) [g/m$^2$] | Rotigotin Reinheit [%] (220 nm / 272 nm) |
|---|---|---|---|---|---|---|---|---|---|
| 1 | 20105025 | groß | - | 15 | PVA / 10 | 9 | 8.88 | 117 | 99.3 / 99.7 |
| 2 | 20105025 | groß | + | 15 | PVA / 10 | 9 | 7.16 *) | 117 | 99.3 / 99.9 |
| 3 | 20105018 | groß | - | 15 | PVA / 10 | 9 | 9.16 | 92 | 99.4 / 100 |
| 4 | 20105018 | groß | + | 15 | PVA / 10 | 9 | 8.36 | 86 | 99.5 / 100 |
| 5 | 20109006 | klein | - | 15 | PVA / 10 | 9 | 8.80 | 82 | 99.6 |
| 6 | 20109007 | klein | - | 15 | PVPVA / 10 | 9 | 8.96 | 98 | 98.3 |
| 7 | 20109008 | klein | - | 15 | PEO/10 | 9 | 7.28 | 88 | 99.1 |
| 8 | 20108030 | groß | + | 15 | - | 25 | 22.43 | 187 | 99.2 / n.d. |
| 9 | 20105045 | klein | - | 15 | - | 25 | 25.2 | 77 | 98.7 / 96.9 |
| PVA=Polyvinylacetat; PEO=Polyethylenoxid; PVPVA=Polyvinylpyrrolidon-Vinylacetat Copolymer | | | | | | | | | |

7. Beispiel: Herstellung EVA-basierter Heißschmelz TTS im Extruder

Die TTS wurden grundsätzlich hergestellt wie in Beispiel 5 beschrieben, wobei die TTS die folgenden Zusammensetzungen hatten: Tabelle 4:

| Lot No (Ch.B.) | Maßstab | Innere Phase / Gehalt [% w/w] | Theoretischer Wirkstoff-gehalt [% w/w] | Gemessener Wirkstoff Gehalt (n=5) [% w/w] | Gewicht (n=10) [g/m$^2$] | Rotigotin Reinheit [%] (220 nm/ 272 nm) |
|---|---|---|---|---|---|---|
| 20103048 | Klein | - | 16.2 | 18.24 | 58 | 98.1/99.7 |
| 20103047 | Klein | - | 16.2 | 15.96 | 127 | 98.8/99.9 |
| 20109019 | Groß | - | 9 | 8.62 | 93 | 98.9/99.9 |
| 20109045 | Groß | - | 15 | 15.08 | 104 | 99.4/n.d. |
| 20109020 | Groß | - | 20 | 18.57 | 89 | 96.1/n.d. |

8. Beispiel: Bestimmung des Wirkstoffflusses im Mäusehautmodell

Für die Fluxmessungen durch Mäusehaut wurde Bauch- und Rückenhaut einer Dicke von ca. 120 bis 150 $\mu$m verwendet. Ein TTS mit einer ausgestanzten Fläche von 2,55 cm$^2$ wird in einer horizontalen Diffusionszelle auf die Hornschichtseite der Bauch- und Rückenhaut haarloser Mäuse fixiert. Unmittelbar anschließend wird die Akzeptorkammer der Zelle mit auf 32°C vortemperierter Phosphat-Pufferlösung (0,066 molar) pH 6,2 luftblasenfrei befüllt und das Freisetzungsmedium auf 32 $\pm$ 0,5°C thermostatisiert.
Zu den Probeentnahmezeiten wird das Freisetzungsmedium gegen frisches, auf 32 $\pm$ 0,5°C thermostatisiertes Medium ausgetauscht. Die Rotigotin-Freisetzung wird per HPLC bestimmt, wie in Beispiel 10 beschrieben.

9. Beispiel: Bestimmung des Rotigotinflusses im Humanhautmodell

Die Bestimmung des Rotigotinfluxes durch Humanhaut wurde im wesentlichen durchgeführt wie in H. Tanojo et al, J. Control Rel. 45 (1997) 41-47 beschrieben.
Hierzu wurde Humanhaut in einer Dicke von ca. 250 $\mu$m aus dem Abdomen gewonnen. Ein TTS mit einer Fläche von Fläche von 2,545 cm$^2$ wurde auf Humanhaut gleicher Fläche aufgebracht, wobei die Haut zur Akzeptorseite hin auf einer Silikonmembran aufliegt (Schema 1). Als Akzeptorphase wurde PBS (0,066 molar) bei PH 6,2 und einer Temperatur von 32$\pm$0.5°C verwendet. Die Experimente wurden mit einem Flux von 5mL/h über 72 Stunden durchgeführt, wobei alle 3 Stunden Proben entnommen wurden. Zu den Probenentnahmezeiten wird das Freisetzungsmedium gegen frisches, auf 32$\pm$0.5°C thermostatiertes Medium ausgetauscht und die Menge des freigesetzten Rotigotins per HPLC gemessen. Die Bestimmung der Fluxrate Q(t) erfolgte bezogen auf die Fläche der Meßzelle (0.552 cm$^2$) gemäß der Formel:

$$Q(t) = \mu g/cm^2 = \text{Konzentration Rotigotin Volumen des Akzeptors}/0.552 \ cm^2$$

Schema 1:

Pflaster, Fläche = 2.545 cm²

Humanhaut, Fläche = 2.545 cm², ~ 250 µm

Silikonmembran, Fläche = 2.545 cm², 150 µm

Diffusionszelle, Fläche = 1.131 cm²
Mit Akzeptor, area = 0.552 cm²

10. Beispiel: Rotigotin-Analytik

(a) Analytik der Wirkstofffreisetzung

Die Messung des Wirkstofffluxes durch Hautpräparate erfolgte per HPLC (Säule RPC18 LichroCART 75-4 Superspher 60select) unter folgenden Bedingungen: 650 Volumenteile (VT) Wasser, 350 VT Acetonitril, 0.5 VT Methansulfonsäure; Raumtemperatur; Wellenlänge: 272 nm; Fluß 2ml

(b) Analytik des Wirkstoffs in der Matrix

(b1) Aufarbeitung der Matrix
Die Klebermatrix wurde mit 0,1 % Methansulfonsäure versetzt, geschüttelt, zentrifugiert und vermessen.
(b2) Analytik des Wirkstoffgehaltes
Der Wirkstoffgehalt wird per isokratischer HPLC unter folgenden Bedingungen bestimmt:

Laufmittel: 65 Volumenteile Wasser mit 0,05 % Methansulfonsäure; 35 Volumenteile Acetonitril mit 0,05 % Methansulfonsäure
Säule: LiChroCART 75 x 4 mm, Superspher 60 RP-select B 5 µm
Flussrate: 2 mUmin, Säulentemperatur: 30°C
UV-Detektion (272 nm)

(b3) Analytik der Wirkstoffstabilität:
Die Reinheit des Rotigotins wurde bestimmt mittels Gradienten-HPLC-methode mit wässriger und organischer (Acetonitril) Phase jeweils mit Zusatz von 0,05 % Methansulfonsäure. Beginn 5 % organischer Anteil, Anstieg über 35 Minuten auf 60 % organischen Anteil.

Säule: LiChrospher 100 CN, 125 mm x 4,6 mm, 5 µm
Flußrate: 1,0 mL, Säulentemperatur 40°C
UV-Detektion (2 Wellenlängen, 272 und 220 nm)

(b4) Bestimmung der dynamischen Viskosität
Die dynamische Viskosität wurde bestimmt wie in RE 36754 beschrieben.

**Patentansprüche**

1. Transdermales Therapeutisches System (TTS) zur transdermalen Verabreichung von Rotigotin, enthaltend eine wirkstoffhaltige Schicht, **dadurch gekennzeichnet, dass**

   (a) die wirkstoffhaltige Schicht

   (1) einen Rotigotinanteil von mindestens 20 Gew% enthält,
   (2) einen Rotigotingehalt von mindestens 2,0 mg/cm$^2$ aufweist,
   (3) optional eine die Wirkstofffreisetzung retardierende Menge eines organischen Wachses und/oder einer inneren Phase-Komponente enthält, und

   (b) nach dem Auftrag des TTS auf der Haut des Patienten Rotigotin über einen Zeitraum von mindestens 5 Tagen in einer steady-state Fluxrate von 100-500 μg/Stunde durch die Haut abgegeben wird.

2. TTS nach Anspruch 1, wobei das TTS eine Rotigotinhaltige Schicht mit einem Rotigontinanteil von mindestens 25 Gew% enthält.

3. TTS nach einem der vorherigen Ansprüche, wobei das TTS eine Rotigotinhaltige Schicht mit einem Rotigontinanteil von mindestens 2,8 mg/cm2 enthält.

4. TTS nach einem der vorherigen Ansprüche, wobei nach dem Auftrag des TTS auf der Haut des Patienten Rotigotin über einen Zeitraum von mindestens 5 Tagen in einer steady-state Fluxrate von 200-300 μg/Stunde durch die Haut abgegeben wird.

5. TTS nach einem der vorherigen Ansprüche, wobei der Weichmacher ein organisches Wachs ist.

6. TTS nach Anspruch 5, wobei der Weichmacher Ceresin oder Ozokerit ist.

7. TTS nach einem der vorherigen Ansprüche, wobei die innere Phase-Komponente ausgewählt ist aus der Gruppe

   a) hydrophiler oder amphiphiler Polymere,
   b) hydrophiler oder amphiphiler Copolymere,
   c) Mischungen aus (a) und/oder (b) mit pharmazeutisch akzeptablen Weichmachern,
   d) Kondensate aus Glycerin und Fettsäuren oder Polyolen,
   e) Geeignete Mischungen aus den Bestandteilen (a)-(d).

8. TTS nach Anspruch 7, wobei die innere Phase-Komponente ausgewählt ist aus der Gruppe Polysaccharide, substituierte Polysaccharide, Polyethylenoxide, Polyvinylacetate, Polyvinylpyrrolidone, Copolymere aus Polyvinylpyrrolidon und (Po-ly)vinylacetat, Polyethylenglycol, Polypropylenglycol, Copolymere von Ethylen und Vinylacetat, Glycerin-Fettsäureester sowie Mischungen aus Polyvinylalkohol mit Glycerin.

Abbildung 1a: Freisetzung durch Mäusehaut (HMS) aus TTS enthaltend 9% (w/w) Rotigotin

EP 1 669 063 A1

Abbildung 1b: WS-Permeation aus Heißschmelz Silikon-TTS enthaltend 25% Rotigotine (WS)

Abbildung 2: Einfluß des Wachsgehaltes auf die Permeation von Rotigotin (WS)

Abbildung 3a: Einfluß des Beladungsgrades auf die Rotigotine-Permeation

Abbildung 3b: Einfluß des Rotigotin-Beladungsgrades auf die Rotigotin-Permeation

EP 1 669 063 A1

Abbildung 4: Einfluß des Matrixgewichtes auf die Rotigotine Permeation

Abbildung 5a: Einfluß des Gehaltes an innerer Phase (PVP) auf die Rotigotine-Permeation

Abbildung 5b: Einfluß des Gehaltes an innerer Phase (PVP) auf die Rotigotine-Permeation

EP 1 669 063 A1

Abbildung 5c: Einfluß des Gehalts an innerer Phase (PEO) auf die Rotigotin-Permeation

EP 1 669 063 A1

Abbildung 6a:  Vergleich der kumulativen Rotigotine-Permeation durch Humanhaut (EHS/SS)

Abb. 6b: Permeation von Rotigotin aus silikonbasiertem Hotmelt-Pflaster
(25 Gew.% Rotigotin) durch Humanhaut

EP 1 669 063 A1

Abb. 6c: Gleichwertigkeit der beiden Hotmeltwachse (25% (w/w) Rotigotine) bezüglich Flux durch Humanhaut im Vergleich zum Phase III Klinikmuster

Abb. 7: Rotigotine-Permeation aus TTS basierend auf verschiedenen Heißschmelzklebern

EP 1 669 063 A1

Abb. 8: Rotigotin-Permeation aus Heißschmelz-Silikon-TTS mit verschiedenen inneren Phasen

Abb. 9: Rotigotine-Permeation durch Mäusehaut aus Heißschmelz-EVA-TTS

Abbildung 9a: Höherbeladenes EVA-hotmelt (20103047; 16.2% (w/w)) durch Humanhaut im Vergleich zum phase III Klinikmuster (WE 11682; 9 % (w/w))

Abbildung 10: Beispielhafter schematischer Aufbau eines TTS

**Europäisches Patentamt**

**EUROPÄISCHER RECHERCHENBERICHT**

Nummer der Anmeldung

EP 06 00 5770

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 99/49852 A (LTS LOHMANN THERAPIE-SYSTEME GMBH; DISCOVERY THERAPEUTICS, INC; MUELLE) 7. Oktober 1999 (1999-10-07) * Ansprüche 1-11 * * Seite 3, Zeile 6 - Seite 9, Zeile 12 * ----- | 1,2,4,5, 7,8 | INV. A61K9/70 A61K31/381 |
| P,X | EP 1 256 340 A (SCHWARZ PHARMA AG; LTS LOHMANN THERAPIE-SYSTEME AG) 13. November 2002 (2002-11-13) * Ansprüche 1-14 * * Absatz [0016] - Absatz [0041] * ----- | 1,4,7,8 | |

RECHERCHIERTE SACHGEBIETE (IPC)

A61K

Der vorliegende Recherchenbericht wurde für alle Patentansprüche erstellt

| Recherchenort | Abschlußdatum der Recherche | Prüfer |
|---|---|---|
| München | 20. April 2006 | Schifferer, H |

**ANHANG ZUM EUROPÄISCHEN RECHERCHENBERICHT**
**ÜBER DIE EUROPÄISCHE PATENTANMELDUNG NR.**

EP 06 00 5770

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-04-2006

| Im Recherchenbericht angeführtes Patentdokument | Datum der Veröffentlichung | Mitglied(er) der Patentfamilie | Datum der Veröffentlichung |
|---|---|---|---|
| WO 9949852 A | 07-10-1999 | AT 210973 T | 15-01-2002 |
| | | AU 746856 B2 | 02-05-2002 |
| | | AU 2934199 A | 18-10-1999 |
| | | BR 9909313 A | 21-11-2000 |
| | | CA 2326630 A1 | 07-10-1999 |
| | | CN 1295470 A | 16-05-2001 |
| | | DE 19814084 A1 | 14-10-1999 |
| | | DK 1033978 T3 | 15-04-2002 |
| | | EP 1033978 A1 | 13-09-2000 |
| | | ES 2170573 T3 | 01-08-2002 |
| | | HK 1031196 A1 | 10-05-2002 |
| | | HU 0101519 A2 | 28-09-2001 |
| | | ID 26646 A | 25-01-2001 |
| | | IL 138722 A | 20-11-2005 |
| | | JP 2002509878 T | 02-04-2002 |
| | | NO 20004915 A | 08-11-2000 |
| | | NZ 507066 A | 31-01-2003 |
| | | PL 343255 A1 | 30-07-2001 |
| | | PT 1033978 T | 28-06-2002 |
| | | SK 14462000 A3 | 09-04-2001 |
| | | TR 200002829 T2 | 22-01-2001 |
| | | TW 579299 B | 11-03-2004 |
| | | US 6884434 B1 | 26-04-2005 |
| | | ZA 200005261 A | 22-05-2001 |
| EP 1256340 A | 13-11-2002 | AT 246919 T | 15-08-2003 |
| | | CN 1462185 A | 17-12-2003 |
| | | DE 60100595 D1 | 18-09-2003 |
| | | DE 60100595 T2 | 24-06-2004 |
| | | DK 1256340 T3 | 01-12-2003 |
| | | WO 02089777 A1 | 14-11-2002 |
| | | EP 1325742 A1 | 09-07-2003 |
| | | ES 2203563 T3 | 16-04-2004 |
| | | HK 1049448 A1 | 12-12-2003 |
| | | HU 0302897 A2 | 29-12-2003 |
| | | JP 2004528359 T | 16-09-2004 |
| | | PT 1256340 T | 31-12-2003 |
| | | SI 1256340 T1 | 31-12-2003 |
| | | ZA 200209980 A | 24-02-2003 |

EPO FORM P0461

Für nähere Einzelheiten zu diesem Anhang : siehe Amtsblatt des Europäischen Patentamts, Nr.12/82